# FASCICULE DE BREVET EUROPEEN

(11) **EP 1 748 043 B1**
(45) Date de publication et mention de la délivrance du brevet: **20.08.2008**
(21) Numéro de dépôt: 06291219.1
(22) Date de dépôt: 27.07.2006
(51) Int. Cl.: C07D 213/65, A61K 31/44, A61P 25/16, A61P 25/26, A61P 25/28, A61P 25/30

(54) **Composés 1,1-pyridinyloxycyclopropanamines polysubstitués, leur procédé de préparation et les compositions pharmaceutiques qui les contiennent**
Polysubstituierte 1,1-pyridinyloxycyclopropanamine Derivate, Verfahren zu ihrer Herstellung und die enthaltenden pharmazeutischen Zusammensetzungen
Polysubstituted 1,1-pyridinyloxycyclopropanamine derivatives, process for their préparation and pharmaceutical compositions containing them

(30) Priorité: 28.07.2005 FR 0508033
(43) Date de publication de la demande: 31.01.2007
(73) Titulaire: Les Laboratoires Servier, 92415 Courbevoie Cedex (FR)
(72) Inventeur: Goldstein, Solo, 92150 Suresnes (FR); Guillonneau, Claude, 92140 Clamart (FR); Charton, Yves, 92330 Sceaux (FR); Lockhart, Brian, 78810 Feucherolles (FR); Lestage, Pierre, 78170 La Celle Saint Cloud (FR)

(56) Documents cités:
- EP-A- 1 170 281

## Description

La présente invention concerne de nouveaux composés 1,1-pyridinyloxy-cyclopropanamines polysubstitués, leur procédé de préparation et les compositions pharmaceutiques qui les contiennent.

Les composés de la présente invention sont particulièrement intéressants d'un point de vue pharmacologique pour leur interaction spécifique avec les récepteurs nicotiniques centraux de type α4β2, trouvant leur application dans le traitement des neuropathologies associées au vieillissement cérébral, des troubles de l'humeur, de la douleur et du sevrage tabagique.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence. Il est largement démontré que parmi les différents neurotransmetteurs, l'acétylcholine tient une place prépondérante dans les fonctions de mémoire et que les voies neuronales cholinergiques sont dramatiquement détruites lors de certaines maladies neurodégénératives ou en déficit d'activation lors du vieillissement cérébral. C'est pourquoi, de nombreuses approches thérapeutiques ont visé à empêcher la destruction du neuromédiateur *via* l'inhibition de l'acétylcholine-estérase ou ont cherché à se substituer au neuromédiateur déficitaire. Dans ce dernier cas, les agonistes cholinergiques proposés ont été de type muscarinique, spécifiques pour les récepteurs post-synaptiques M1.

Récemment, il a été montré que l'atteinte cholinergique liée à la maladie d'Alzheimer touchait davantage les neurones portant les récepteurs nicotiniques que ceux portant les récepteurs muscariniques (Schroder et Coll., « Alzheimer disease : therapeutic strategies », Birkhauser Boston, 1994, 181-185). De plus, de nombreuses études ont démontré que la nicotine possède des propriétés facilitatrices de la mémoire (Prog. Neuropsychopharmacol., 1992, 16, 181-191) et que ces propriétés s'exercent tout autant sur les fonctions mnésiques (Psychopharmacol., 1996, 123, 88-97) que sur les facultés d'attention et de vigilance (Psychopharmacol., 1995, 118, 195-205). Par ailleurs, la nicotine exerce des effets neuroprotecteurs vis-à-vis d'agents excitotoxiques tel que le glutamate (Brain Res., 1994, 644, 181-187).

L'ensemble de ces données est très probablement à relier avec les études épidémiologiques qui ont montré une moindre incidence de maladie d'Alzheimer ou de Parkinson chez les sujets fumeurs. De plus, plusieurs études ont montré l'intérêt de la nicotine dans le traitement des troubles de l'humeur tels que les états dépressifs, anxieux ou schizophréniques. Enfin, il a été montré que la nicotine possède des propriétés antalgiques. L'ensemble des propriétés thérapeutiques de la nicotine, ainsi que celles décrites pour d'autres agents nicotiniques, est sous tendu par une activité vis-à-vis de récepteurs centraux qui diffèrent structurellement et pharmacologiquement des récepteurs périphériques (muscle et ganglion). Les récepteurs centraux de type α4β2 sont les plus représentés dans le système nerveux central et ont été impliqués dans la plupart des effets thérapeutiques de la nicotine (Life Sci., 1995, 56, 545-570).

Plusieurs documents tels que Synlett., 1999, 7, 1053-1054 ; J. Med. Chem, 1985, 28(12), 1953-1957 et 1980, 23(3), 339-341 ; 1970, 13(5), 820-826 ; 1972, 15(10), 1003-1006 ; J. Am. Chem. Soc., 1987, 109(13), 4036-4046, ou quelques brevets ou demandes de brevets comme DE 36 08 727, EP 124 208 ou WO 94/10158 décrivent et revendiquent des composés comportant un motif cyclopropanique 1,1 ou 1,2-disubstitué. Aucunes de ces références ne décrivent ou ne suggèrent pour ces composés une activité pharmacologique spécifique vis-à-vis des récepteurs nicotiniques et plus particulièrement vis-à-vis des récepteurs nicotiniques centraux de type α4β2, propriété originale des composés décrits par la Demanderesse. La demande de brevet EP 1 170 281 décrit des composés cyclopropaniques 1,1 et 1,2-disubstitués qui sont des ligands nicotiniques.

Les composés de la présente invention sont donc nouveaux et constituent de puissants ligands nicotiniques sélectifs du sous-type réceptoriel α4β2 central. De ce fait, ils sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur.

Plus particulièrement, la présente invention concerne les composés de formule (I) : 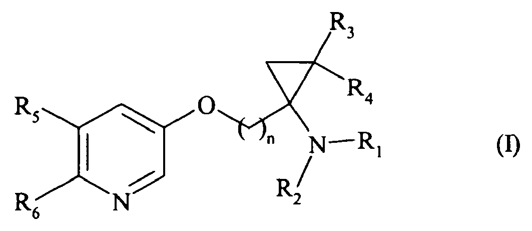 dans laquelle :
- n: représente un nombre entier compris entre 1 et 6 inclus,
- R₁ et R₂,: identiques ou différents, indépendamment l'un de l'autre, représentent un atome d'hydrogène, ou un groupement alkyle (C₁-C₆) linéaire ou ramifié ou arylalkyle (C₁-C₆) linéaire ou ramifié,
- R₃ et R₄,: identiques ou différents, indépendamment l'un de l'autre, représentent un atome d'hydrogène, ou un groupement alkyle (C₁-C₆) linéaire ou ramifié, étant entendu qu'au moins un des deux groupements R₃ ou R₄ représente un groupement alkyle (C₁-C₆) linéaire ou ramifié,
- R₅ et R₆,: identiques ou différentes, indépendamment l'un de l'autre, représentent un atome d'hydrogène, ou un groupement alkyle (C₁-C₆) linéaire ou ramifié, halogène, hydroxy, alkoxy (C₁-C₆) linéaire ou ramifié, cyano, nitro, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, ou amino éventuellement substitué par un ou deux groupements alkyle (C₁-C₆) linéaire ou ramifié,
par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi halogène, alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino éventuellement substitué par un ou deux groupements alkyle (C₁-C₆) linéaire ou ramifié.

Des composés préférés de l'invention sont les composés pour lesquels n est un entier prenant la valeur 1.

Les substituants R₁ et R₂ préférés selon l'invention sont l'atome d'hydrogène et le groupement alkyle (C₁-C₆) linéaire ou ramifié.

Encore plus préférentiellement, les substituants R₁ et R₂ préférés selon l'invention sont l'atome d'hydrogène et le groupement méthyl.

Les substituants R₃ et R₄ préférés selon l'invention sont l'atome d'hydrogène et le groupement méthyl.

Les substituants R₅ et R₆ préférés selon l'invention sont l'atome d'hydrogène et l'atome d'halogène.

D'une façon avantageuse, les composés préférés de l'invention sont les composés pour lesquels R₅ représente un atome d'halogène et R₆ représente un atome d'hydrogène.

D'une façon encore plus avantageuse, les composés préférés de l'invention sont les composés pour lesquels R₅ représente un atome d'hydrogène et R₆ représente un atome d'halogène.

La notation (1*S,*2*S*), (1*R*,2*R*) suivi du nom du composé signifie que le produit obtenu est un mélange racémique et donc, que les deux configurations sont possibles.
A titre d'exemple :
(1*S*,2*S*), (1*R*,2*R*)-2-Méthyl-1-[(3-pyridinyloxy)méthyl]cyclopropanamine signifie que le produit obtenu, le mélange racémique, contient le (1*S*,2*S*)-2-méthyl-1-[(3-pyridinyloxy) méthyl]cyclopropanamine et le (1*R*,2*R*)-2-éthyl-1-[(3-pyridinyloxy)méthyl]cyclopropanamine

La notation (*R ou S*) suivi du nom du composé signifie que le produit obtenu est un énantiomère optiquement pur. La présence de (-) et/ou (+) indique le signe du pouvoir rotatoire.

La notation (*R,S*) suivi du nom du composé signifie que le produit obtenu est un mélange racémique et donc, que les deux configurations sont possibles.

La notation (1*S*,2*S*) ou (1*R*,2*R*) suivi du nom du composé signifie que le produit obtenu est un énantiomère optiquement pur. La présence de (-) et/ou (+) indique le signe du pouvoir rotatoire.
A titre d'exemple :
Dichlorhydrate de (1*S*,2*S*) ou (1*R*,2*R*)-(-)-*N*,2-diméthyl-1-[(3-pyridinyloxy)méthyl] cyclopro-panamine singifie que le produit obtenu, l'énantiomère optiquement pur, est le dichlorhydrate de (1*S*,2*S*)-(-)-*N*,2-diméthyl-1-[(3-pyridinyloxy)méthyl]cyclopropanamine ou le dichlorhydrate de (1*R*,2*R*)-(-)-*N*,2-diméthyl-1-[(3-pyridinyloxy)méthyl] cyclopropanamine

Par énantiomère α et énantiomère β, on entend les énantiomères optiquement purs du mélange racémique correspondant.

D'une façon particulièrement avantageuse, les composés préférés de l'invention sont le :
(1*S*,2*S*), (1*R*,2*R*)-*N*,2-diméthyl-1-[(3-pyridinyloxy)méthyl]cyclopropanamine
(1*S*,2*S*) ou (1*R*,2*R*)-(-)-*N*,2-diméthyl-1-[(3-pyridinyloxy)méthyl]cyclopropanamine
(1*S*,2*S*) ou (1*R*,2*R*)-(+)-*N*,2-diméthyl-1-[(3-pyridinyloxy)méthyl]cyclopropanamine
(1*S,*2*S*), (1*R*,2*R*)-2-Méthyl-1-[(3-pyridinyloxy)méthyl]cyclopropanamine
(1*S*,2*S*) ou (1*R*,2*R*)-(-)-2-méthyl-1-[(3-pyridinyloxy)méthyl]cyclopropanamine
(1*S*,2*S*) ou (1*R*,2*R*)-(+)-2-méthyl-1-[(3-pyridinyloxy)méthyl]cyclopropanamine
(1*S*,2*S*), (1*R*,2*R*)-*N*,*N*,2-Triméthyl-1-[(3-pyridinyloxy)méthyl]cyclopropanamine
(1*S*,2*S*) ou (1*R*,2*R*)-(+)-*N*,*N*,2-triméthyl-1-[(3-pyridinyloxy)méthyl]-cyclopropanamine
(1*S*,2*S*) ou (1*R*,2*R*)-(-)-*N*,*N*,2-triméthyl-1-[(3-pyridinyloxy)méthyl]-cyclopropanamine
(1*S,*2*S),* (1*R*,2*R*)-1-{[(6-Chloro-3-pyridinyl)oxy]méthyl}-*N*,2-diméthylcyclopropanamine
(1*S,2S*) ou (1*R*,2*R*)-(-)-1-{[(6-chloro-3-pyridinyl)-oxy]méthyl}-*N*,2-diméthylcyclopropanamine
(1*S*,2*S*) ou (1*R*,2*R*)-(+)-1-{[(6-chloro-3-pyridinyl)-oxy]méthyl}-*N*,2-diméthylcyclopro-panamine
(1*S*,2*S*), (1*R*,2*R*)-1-{[(6-Chloro-3-pyridinyl)oxy]méthyl}-2-méthylcyclopropanamine
(1*S*,2*S*) ou (1*R*,2*R*)-(-)-1-{[(6-chloro-3-pyridinyl)-oxy]méthyl}-2-méthylcyclopropanamine
(1*S*,2*S*) ou (1*R*,2*R*)-(+)-1-{[(6-chloro-3-pyridinyl)-oxy]méthyl}-2-méthylcyclopro-panamine
(1*S*,2*S*), (1*R*,2*R*)-1-{[(6-Chloro-3-pyridinyl)oxy]méthyl}-*N*,*N*,2-triméthylcyclopro-panamine
(1*S*,2*S*) ou (1*R*,2*R*)-(+)-1-{[(6-chloro-3-pyridinyl)-oxy]méthyl}-*N*,*N*,2-triméthylcyclopro-panamine
(1*S*,2*S*) ou (1*R*,2*R*)-(-)-1-{[(6-chloro-3-pyridinyl)-oxy]méthyl}-*N*,*N*,2-triméthylcyclopro-panamine
(1*S*,2*R*), (1*R*,2*S*)-*N*,2-Diméthyl-1-[(3-pyridinyloxy)méthyl]-cyclopropanamine
(1*S*,2*R*) ou (1*R*,2*S*)-(+)-*N*,2-diméthyl-1-[(3-pyridinyloxy)méthyl]cyclopropanamine
(1*S*,2*R*) ou (1*R*,2*S*)-(-)-*N*,2-diméthyl-1-[(3-pyridinyloxy)méthyl]cyclopropanamine
(1*S*,2*R*), (1*R*,2*S*)-1-{[(5-bromo-3-pyridinyl)oxy]méthyl}-*N*,2-diméthyloyolopropanamine
(1*S*,2*R*), (1*R*,2*S*)-2-méthyl-1-[(3-pyridinyloxy)méthyl]cyclopropanamine
(1*S*,2*R*), (1*R*,2*S*)*-N*,*N*,2-triméthyl-1-[(3-pyridinyloxy)méthyl]cyclopropanamine
(*R*,*S*)-*N*,2,2-triméthyl-1-[(3-pyridinyloxy)méthyl]cyclopropanamine
(R ou *S*)-(+)-*N*,2,2-triméthyl-1-[(3-pyridinyloxy)-méthyl]cyclopropanamine
(*R* ou *S*)-(-)-*N*,2,2-triméthyl-1-[(3-pyridinyloxy)-méthyl]cyclopropanamine

Les isomères ainsi que les sels d'addition à un acide ou à une base pharmaceutiquement acceptable des composés préférés font partie intégrante de l'invention.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique.

Parmi les bases pharmaceutiquement acceptables, on peut citer l'hydroxyde de sodium, l'hydroxyde de potassium, la triéthylamine, la tertbutylamine.

La présente invention concerne également le procédé de préparation des composés de formule (I), caractérisé en ce qu'on utilise comme produit de départ un composé de formule (II) : 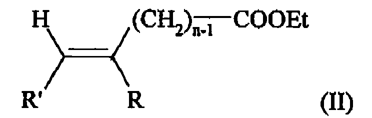 dans lequel R représente un groupement choisi parmi éthoxycarbonyle, cyano et isocyano, R' représente un groupement choisi parmi méthyle et 2-bromopropyle, composé de formule (II), qui sont mis à réagir avec de l'iodure de triméthylsulfoxonium en présence d'hydrure de sodium dans du diméthyle sulfoxyde, pour conduire aux composés de formule (III) : 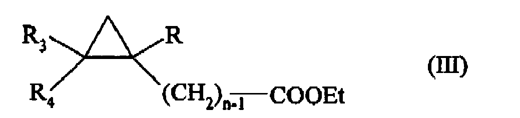 dans laquelle R est tel que défini précédemment et R₃, R₄ et n sont tels que définis dans la formule (I),
composés de formule (III), qui sont :
- soit mis en présence d'hydroxyde de sodium dans l'éthanol lorsque R représente un groupement éthoxycarbonyle, pour conduire aux composés de formule (IV) : 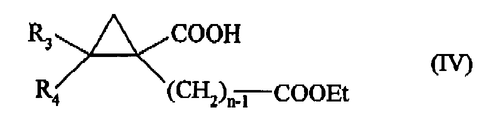
dans laquelle R₃, R₄ et n sont tels que définis précédemment,
composés de formule (IV), qui sont mis à réagir avec du diphénylphosphorylazide et du tert-butanol dans le toluène, en présence de triéthylamine, pour conduire aux composés de formule (V) : 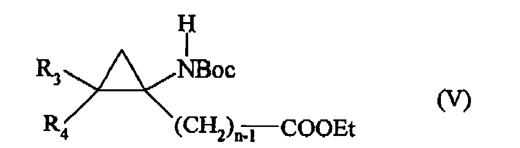 dans laquelle R₃, R₄ et n sont tels que définis précédemment et Boc représente le groupement tert-butoxycarbonyl, composés de formule (V) qui est mis à réagir avec un composé de formule (VI) :

R'₁-L₁ (VI)

dans laquelle R'₁ représente un groupement choisi parmi alkyle (C₁-C₆) linéaire ou ramifié et arylalkyle (C₁-C₆) linéaire ou ramifié, et L₁ représente un groupement partant usuel de la chimie organique, en présence de tert-butylate de potassium dans du diméthylformamide, pour conduire aux composés de formule (VII) : 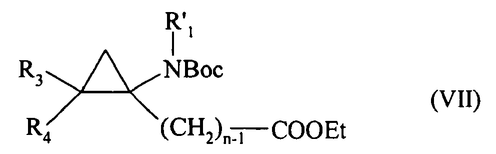 dans laquelle R₃, R₄, R'₁, Boc et n sont tels que définis précédemment,
l'ensemble des composés de formules (V) et (VII) formant les composés de formule (VIII) : 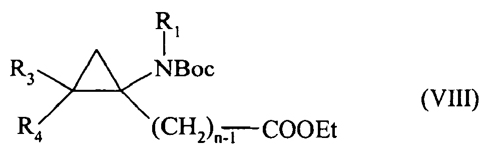 dans laquelle R₃, R₄, Boc et n sont tels que définis précédemment et R₁ est tel que défini dans la formule (I),
composés de formule (VIII), qui sont :
* soit mis en présence d'un agent réducteur dans un solvant anhydre, pour conduire aux composés de formule (IX) : 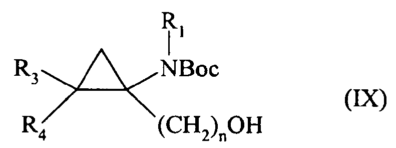
dans laquelle R₃, R₄, Boc, n et R₁ sont tels que définis précédemment,
composés de formule (IX) qui sont soit mis à réagir avec du triphénylphosphine et du tétrabromométhane dans l'éther, pour conduire aux composés de formule (X) : 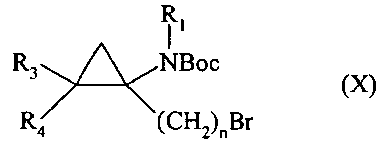 dans laquelle R₃, R₄, Boc, n et R₁ sont tels que définis précédemment,
composés de formule (X) qui sont mis à réagir avec un composé de formule (XI) : 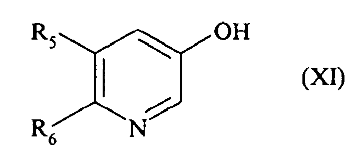 dans lequel R₅ et R₆ sont tels que définis dans la formule (I), en présence de carbonate de césium dans la butanone, pour conduire aux composés de formule (XII) : 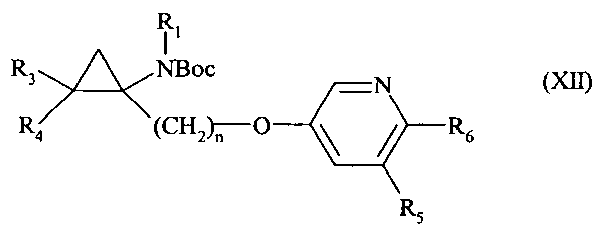 dans laquelle R₃, R₄, R₅, R₆, R₁, n et Boc sont tels que définis précédemment,
composés de formule (XII) qui est mis en présence d'acide chlorhydrique dans le dioxanne pour conduire aux composés de formule (I/a), cas particulier des composés de formule (I) : 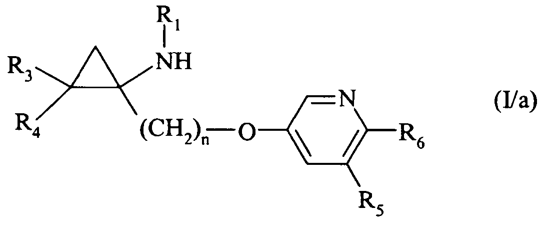 dans laquelle R₁, R₃, R₄, R₅, R₆ et n sont tels que définis précédemment,
composés de formule (I/a) qui sont :
- soit traités par de l'acide formique et une solution aqueuse de formaldéhyde, pour conduire aux composés de formule (I/b), cas particulier des composés de formule (I) : 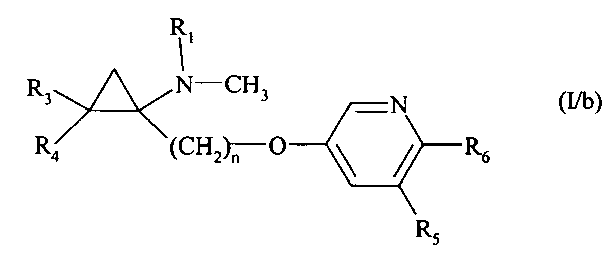
dans laquelle R₁, R₃, R₄, R₅, R₆ et n sont tels que définis précédemment,
- soit mis à réagir avec un composé de formule (XIII) :

   R'₂ -L₁ (XIII)

   dans laquelle L₁ est tel que défini précédemment et R'₂ représente un groupement choisi parmi alkyle (C₁-C₆) linéaire ou ramifié et arylalkyle (C₁-C₆) linéaire ou ramifié, dans les mêmes conditions que les composés de formule (V), pour conduire aux composés de formule (I/c), cas particulier des composés de formule (I) : 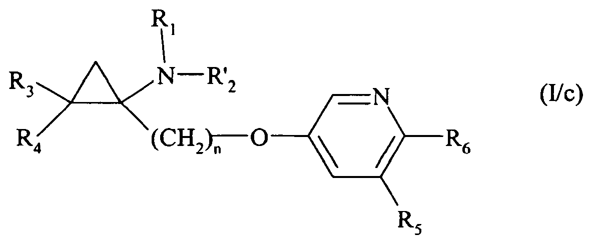
dans laquelle R₁, R₃, R₄, R₅, R₆, n et R'₂ sont tels que définis précédemment,
ou composés de formule (VIII) qui sont :
* soit traités par de l'acide formique et une solution aqueuse de formaldéhyde pour conduire aux composés de formule (XIV) : 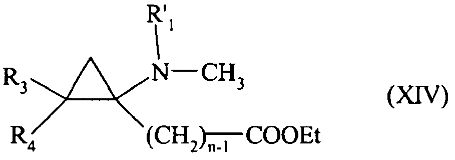
dans laquelle R'₁, R₃, R₄ et n sont tels que définis précédemment,
* soit successivement :
   - traités par de l'acide chlorhydrique dans du dioxanne
   - puis par un composé de formule (XIII) tel que définis précédemment, dans les mêmes conditions que les composés de formule (V) pour conduire aux composés de formule (XV) : 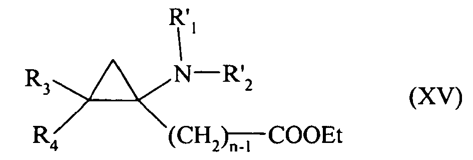
dans laquelle R'₁, R'₂, R₃, R₄ et n sont tels que définis précédemment,
composés de formule (XIV) et (XV) qui sont mis en présence d'un agent réducteur dans un solvant anhydre pour conduire aux composés de formule (XVI) : 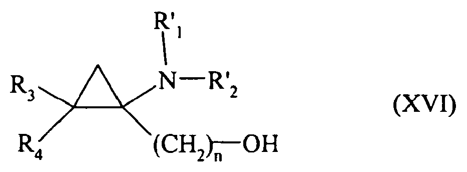 dans laquelle R'₁, R'₂, R₃, R₄ et n sont tels que définis précédemment,
composés de formule (XVI) qui sont mis à réagir avec un composé de formule (XVII) : 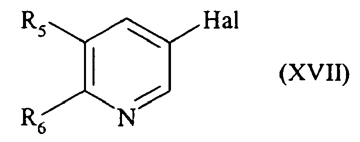 dans lequel R₅ et R₆ sont tels que définis précédemment, et Hal représente un atome de brome ou de fluor, en présence de tert-butylate de potassium dans du DMSO, pour conduire aux composés de formule (I/d), cas particulier des composés de formule (I) : 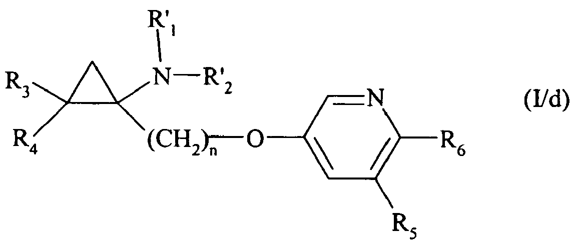 dans laquelle R'₁, R'₂, R₃, R₄, R₅, R₆ et n sont tels que définis précédemment,
composés de formule (I/d) qui est mis à réagir avec de l'acide chlorhydrique en présence d'hydroxyde de palladium dans de l'éthanol lorsque R'₁ représente un groupement benzyle, pour conduire aux composés de formule (I/e), cas particulier des composés de formule (1) : 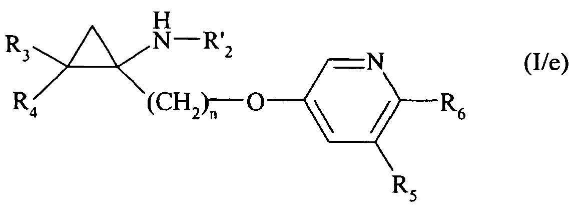 dans laquelle R'₂, R₃, R₄, R₅, R₆ et n sont tels que définis précédemment,
ou composés de formule (VIII) qui sont :
* soit successivement :
   - traités par de l'acide chlorhydrique dans l'éthanol
   - puis une solution aqueuse d'hydrogénocarbonate de sodium pour conduire aux composés de formule (XVIII) : 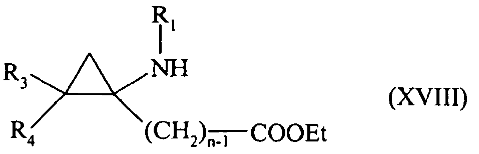
dans laquelle R₁, R₃, R₄ et n sont tels que définis précédemment,
composés de formule (XVIII) qui sont mis à réagir avec du chlorure de benzoyle en présence de triéthylamine dans du tétrahydrofuranne, pour conduire aux composés de formule (XIX) : 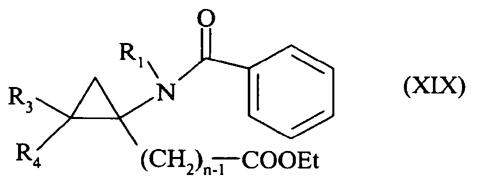 dans laquelle R₁, R₃, R₄ et n sont tels que définis précédemment,
composés de formule (XIX) qui est mis en présence d'un agent réducteur dans un solvant anhydre pour conduire aux composés de formule (XX) : 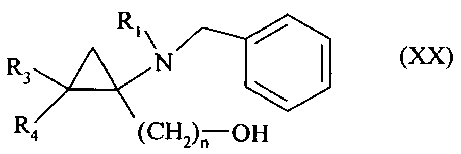 dans laquelle R₁, R₃, R₄ et n sont tels que définis précédemment,
composés de formule (XX) qui est soumis aux mêmes conditions de réaction que les composés de formules (XVI), pour conduire aux composés de formule (1/f), cas particulier des composés de formule (1) : 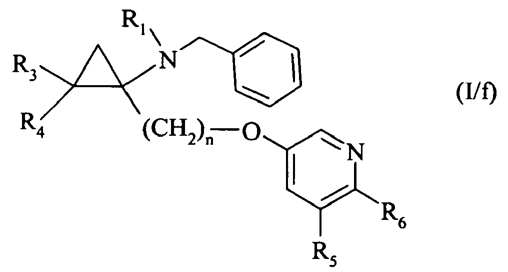 dans laquelle R₁, R₃, R₄, R₅, R₆ et n sont tels que définis précédemment,
composés de formule (I/f) qui sont soumis aux mêmes conditions de réaction que les composés de formule (I/d), pour conduire aux composés de formule (I/a) tel que défini précédemment, cas particulier des composés de formule (I),
ou composés de formule (IX) qui sont :
- soit mis à réagir avec de l'acide chlorhydrique dans du dioxanne, lorsque R₁ représente l'atome d'hydrogène, pour conduire aux composés de formule (XXI) : 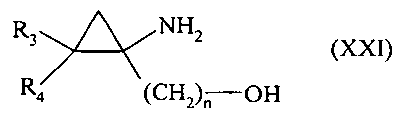
dans laquelle R₃, R₄ et n sont tels que définis précédemment,
composés de formule (XXI) qui sont soumis aux mêmes conditions de réaction que les composés de formule (XVI), pour conduire aux composés de formule (I/g), cas particulier des composés de formule (I), composés de formule (I/g) de stéréochimie cis de (CH₂)ₙ par rapport à l'un des substituants R₃ ou R₄ qui représente un alkyle, l'autre substituant R₃ ou R₄ représente un atome d'hydrogène : 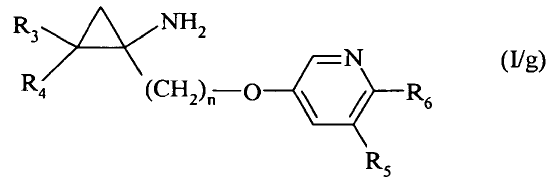 dans laquelle R₃, R₄, R₅, R₆ et n sont tels que définis précédemment,
ou composés de formule (III) qui sont :
- soit mis à réagir avec de l'hydrure d'aluminium lithium dans de l'éther, lorsque R représente un groupement isocyano, pour conduire aux composés de formule (XXII) : 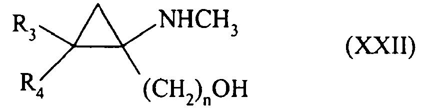
dans laquelle R₃, R₄ et n sont tels que définis précédemment,
composés de formule (XXII) qui sont mis à réagir avec un composé de formule (XVII) tel que défini précédemment, dans les mêmes conditions que les composés de formule (XVI) pour conduire aux composés de formule (I/h), cas particulier des composés de formule (I) : 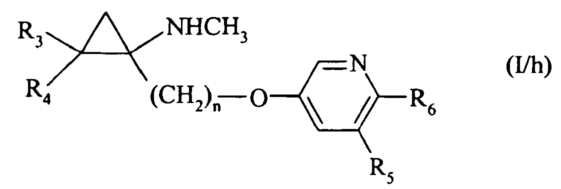 dans laquelle R₃, R₄, R₅, R₆ et n sont tels que définis précédemment,
- soit mis à réagir avec du borohydrure de sodium dans du tétrahydrofuranne, lorsque R représente un groupement cyano, pour conduire aux composés de formule (XXIII) : 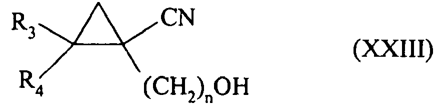
dans laquelle R₃, R₄ et n sont tels que définis précédemment,
composés de formule (XXIII) qui sont mis aux mêmes conditions de réaction que les composés de formule (XVI) pour conduire aux composés de formule (XXIV) : 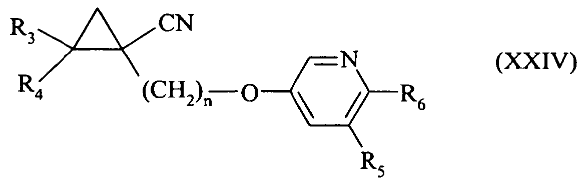 dans laquelle R₃, R₄, R₅, R₆ et n sont tels que définis précédemment,
composés de formule (XXIV) qui sont mis en présence d'une solution aqueuse d'eau oxygénée et d'hydroxyde de lithium dans l'éthanol pour conduire aux composés de formule (XXV) : 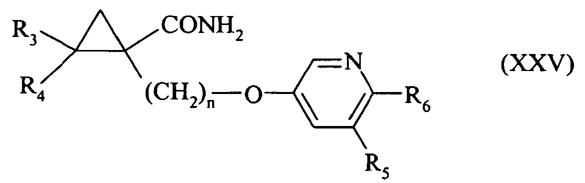 dans laquelle R₃, R₄, R₅, R₆ et n sont tels que définis précédemment,
composés de formule (XXV) qui sont mis à réagir avec du brome et de l'hydroxyde de potassium dans de l'eau, pour conduire aux composés de formule (I/i), cas particulier des composés de formule (I), composés de formule (I/i) de stéréochimie cis de NH₂ par rapport à l'un des substituants R₃ ou R₄ qui représente un alkyle, l'autre substituant R₃ ou R₄ représente un atome d'hydrogène : 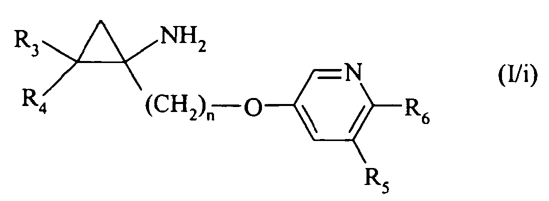 dans laquelle R₃, R₄, R₅, R₆ et n sont tels que définis précédemment,
composés de formule (I/i) qui sont soumis aux mêmes conditions de réaction que les composés de formule (I/a) pour conduire aux composés de formule (I/j), cas particulier des composés de formule (I) : 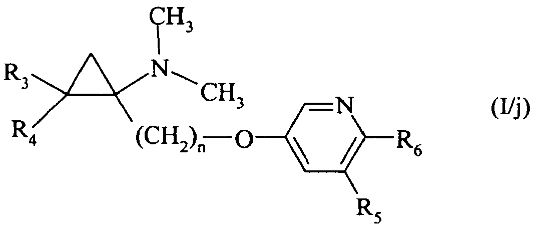 dans laquelle R₃, R₄, R₅, R₆ et n sont tels que définis précédemment,
l'ensemble des composés de formule (I/a) à (I/j) formant l'ensemble des composés de l'invention qui sont purifiés, le cas échéant, selon des techniques classiques de purification, qui peuvent être séparés en leurs différents isomères, selon une technique classique de séparation et qui sont transformés, le cas échéant, en leurs sels d'addition à un acide ou à une base pharmaceutiquement acceptable.

Les composés de formule (II), (VI), (XI), (XIII) et (XVII) sont soit des produits commerciaux, soit obtenus selon des méthodes classiques de la synthèse organique, bien connues de l'homme de l'art.

Selon une variante de l'invention, les composés de formule (I), dans le cas où l'un des substituants R₃ ou R₄ représente un alkyle (C₁-C₆) linéaire ou ramifié et l'autre substituant R₃ ou R₄ représente un atome d'hydrogène, de stéréochimie cis de (CH₂)ₙ par rapport au substituant R₃ ou R₄ qui représente un alkyle de formule (I/₁) : 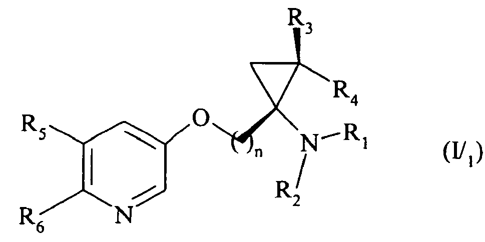 dans laquelle R₃ représente un groupement alkyle (C₁-C₆) linéaire ou ramifié, R₄ représente un atome d'hydrogène et R₁, R₂, R₅, R₆ et n sont tels que définis dans la formule (I),
peuvent être préparés à partir d'un composé de formule (III/₁) : 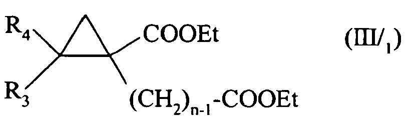 dans laquelle R₃, R₄ et n sont tels que définis précédemment, qui sont mis en présence d'hydroxyde de sodium dans l'éthanol pour conduire aux composés de formule (IV/₁) : 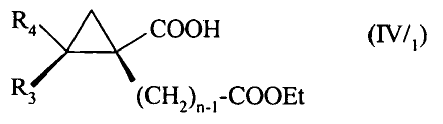 dans laquelle R₃, R₄ et n sont tels que définis précédemment,
composés de formule (IV/₁), qui sont mis à réagir avec du diphénylphosphorylazide et du tert-butanol dans le toluène, en présence de triéthylamine, pour conduire aux composés de formule (V/₁) : 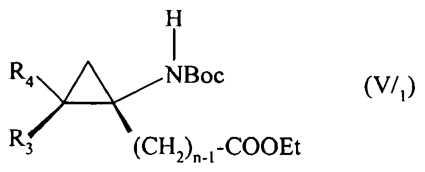 dans laquelle R₃, R₄, n et Boc sont tels que définis précédemment, composés de formule (V/₁) qui sont mis à réagir avec un composé de formule (VI) tel que défini précédemment, en présence de tert-butylate de potassium dans du diméthylformamide pour conduire aux composés de formule (VI/₁) : 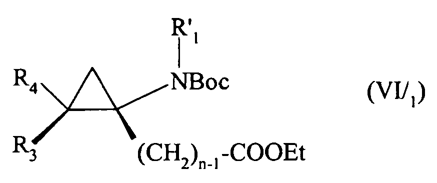 dans laquelle R₃, R₄, n, R'₁ et Boc sont tels que définis précédemment,
l'ensemble des composés de formules (V/₁) et (VI/₁) formant les composés de formule (VII/₁) : 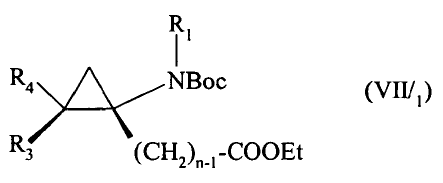 dans laquelle R₁, R₃, R₄, n et Boc sont tels que définis précédemment,
les composés de formule (VII/₁) qui sont :
- *soit* traités par de l'acide formique et une solution aqueuse de formaldéhyde pour conduire aux composés de formule (VIII/₁) : 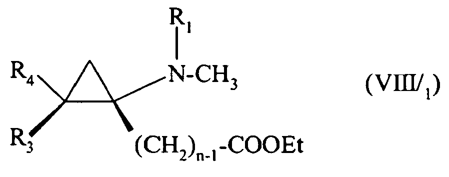
dans laquelle R₁, R₃, R₄ et n sont tels que définis précédemment,
- *soit* traités successivement par de l'acide chlorhydrique dans le dioxane puis par un composé de formule (XIII), tel que défini précédemment, en présence de tert-butylate de potassium dans du diméthylformamide pour conduire aux composés de formule (IX/₁) : 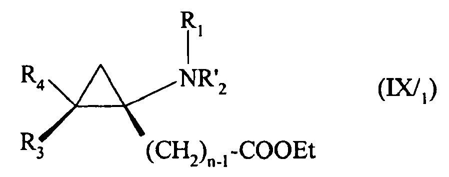
dans laquelle R₁, R₃, R₄, n et R'₂ sont tels que définis précédemment,
les composés de formules (VII/₁), (VIII/₁) et (IX/₁) qui sont mis en présence d'un agent réducteur dans un solvant anhydre pour conduire aux composés de formule (X/₁) : 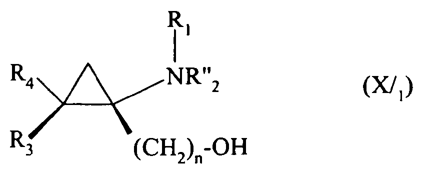 dans laquelle R"₂ représente un groupement Boc, CH₃ et R'₂ et R₁, R₃, R₄ et n sont tels que définis précédemment,
les composés de formule (X/₁) qui sont :
* soit, lorsque R"₂ représente un groupement Boc, mis en présence d'acide chlorhydrique dans du dioxane pour conduire aux composés de formule (XI/₁) : 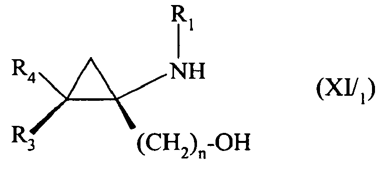
dans laquelle R₁, R₃, R₄ et n sont tels que définis précédemment, les composés de formules (X/₁) et (XI/₁) forment les composés de formule (XII/₁) : 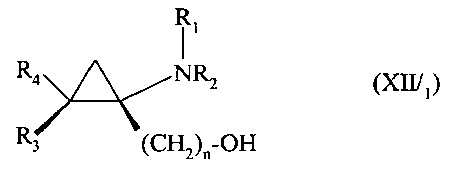 dans laquelle R₁, R₂, R₃, R₄ et n sont tels que définis précédemment,
les composés de formule (XII/₁) qui sont mis à réagir avec les composés de formule (XVII), tel que défini précédemment, en présence de tert-butylate de potassium dans du DMSO, pour conduire aux composés de formule (I/a₁), cas particulier des composés de formule (I) : 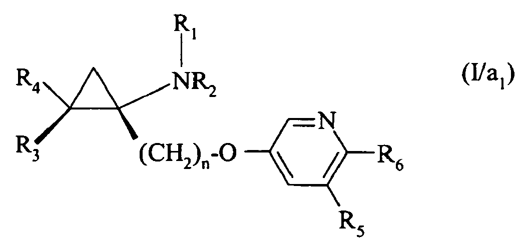 dans laquelle R₁, R₂, R₃, R₄, R₅, R₆ et n sont tels que définis précédemment,
les composés de formule (I/a₁) sont purifiés par HPLC chirale puis salifiés par une solution éthanolique d'acide chlorhydrique pour obtenir les sels chlorés des deux énantiomères (-) et (+) correspondants,
* soit mis en présence de triphénylphosphine et du tétrabromométhane dans de l'éther pour conduire aux composés de formule (XIII/₁) : 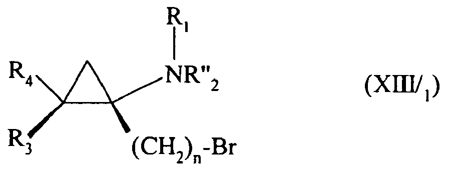
dans laquelle R₁, R₃, R₄, R"₂ et n sont tels que définis précédemment,
composés de formule (XIII/₁) qui sont mis à réagir avec le composé de formule (XI), tel que défini précédemment, en présence de carbonate de césium dans le butanone pour conduire aux composés de formule (XIV/₁) : 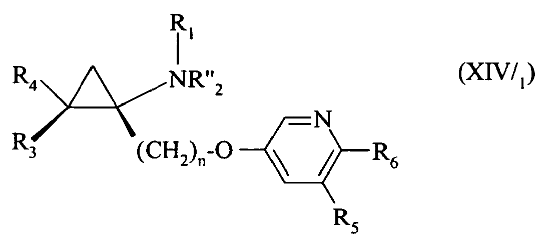 dans laquelle R₁, R₃, R₄, R₅, R₆, R"₂ et n sont tels que définis précédemment,
composés de formule (XIV/₁) qui, lorsque R"₂ représente un groupement Boc, sont mis en présence d'acide chlorhydrique dans du dioxane pour conduire aux composés de formule (I/b₁), cas particulier des composés de formule (I) : 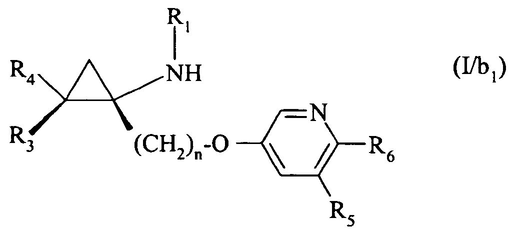 dans laquelle R₁, R₃, R₄, R₅, R₆ et n sont tels que définis précédemment,
les composés de formule (I/b₁) sont purifiés dans les mêmes conditions que les composés de formule (I/a₁),
les composés de formules (I/a₁) et (I/b₁) avant purification pouvant éventuellement, lorsque R₁ et/ou R₂ représente un atome d'hydrogène, être traités par de l'acide formique et une solution aqueuse de formaldéhyde pour conduire aux composés de formules (I/c₁), (I/c₁) et (I/c"₁), cas particuliers des composés de formule (I) : 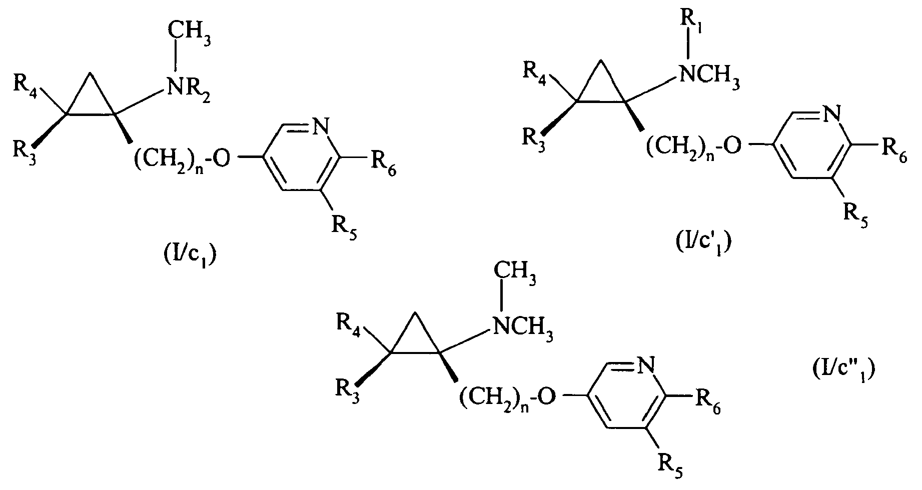 dans lesquelles R₁, R₂, R₃, R₄, R₅, R₆ et n sont tels que définis précédemment,
les composés de formules (I/c₁), (I/c'₁) et (I/c"₁) sont purifiés dans les mêmes conditions que les composés de formule (I/a₁).

Selon une autre variante de l'invention, les composés de formule (I), dans le cas où l'un des substituants R₃ ou R₄ représente un alkyle (C₁-C₆) linéaire ou ramifié et l'autre substituant R₃ ou R₄ représente un atome d'hydrogène, de stéréochimie cis de NR₁R₂ par rapport au substituant R₃ ou R₄ qui représente un alkyle de formule (I/₂) : 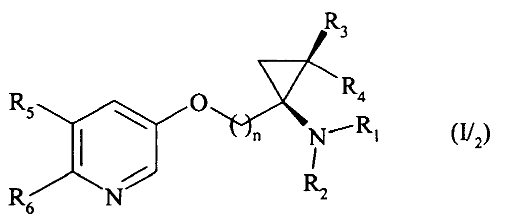 dans laquelle R₃ représente un groupement alkyle (C₁-C₆) linéaire ou ramifié, R₄ représente un atome d'hydrogène et R₁, R₂, R₅, R₆ et n sont tels que définis dans la formule (I),
peuvent être préparés à partir d'un composé de formule (II/₂) : 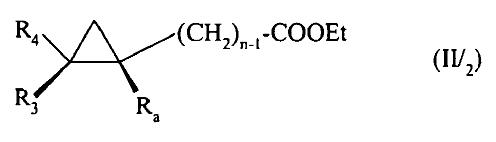 dans laquelle Rₐ représente un groupement cyano ou isocyano et R₃, R₄ et n sont tels que définis précédemment, qui sont :
- **soit** mis en présence d'un agent réducteur dans un solvant anhydre lorsque Rₐ représente un groupement isocyano, pour conduire aux composés de formule (III/₂) : 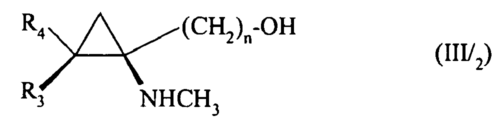
dans laquelle R₃, R₄ et n sont tels que définis précédemment, composés de formule (III/₂) qui sont mis à réagir avec le composé de formule (XVII), tel que défini précédemment, en présence d'une base forte dans un solvant anhydre, pour conduire aux composés de formule (I/a₂), cas particulier des composés de formule (I) : 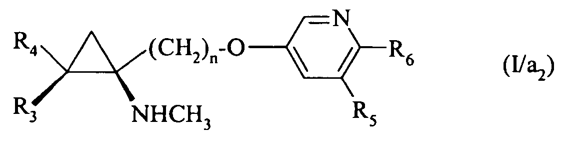 dans laquelle R₃, R₄, R₅, R₆ et n sont tels que définis précédemment,
- **soit** mis en présence de borohydrure de sodium dans un mélange eau/tétrahydrofurane lorsque Rₐ représente un groupement cyano, pour conduire aux composés de formule (IV/₂): 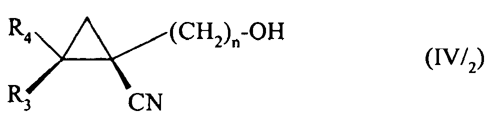
dans laquelle R₃, R₄ et n sont tels que définis précédemment, composés de formule (IV/₂) qui sont mis à réagir avec le composé de formule (XVII), tel que défini précédemment, en présence d'une base forte dans un solvant anhydre, pour conduire aux composés de formule (V/₂) : 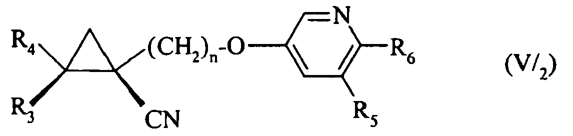 dans laquelle R₃, R₄, R₅, R₆ et n sont tels que définis précédemment, les composés de formule (V/₂) sont mis en présence d'hydroxyde de lithium et d'eau oxygénée dans l'éthanol, pour conduire aux composés de formule (VI/₂) : 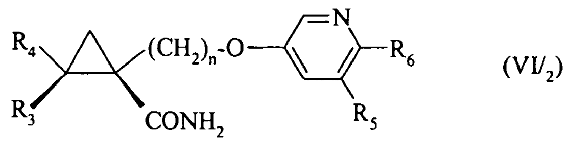 dans laquelle R₃, R₄, R₅, R₆ et n sont tels que définis précédemment, composés de formule (VI/₂) qui sont mis en présence d'hydroxyde de potassium et de brome dans l'eau pour conduire aux composés de formule (I/b₂), cas particuliers des composés de formule (I) : 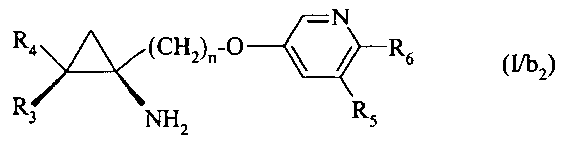 dans laquelle R₃, R₄, R₅, R₆ et n sont tels que définis précédemment, les composés de formules (I/a₂) et (I/b₂) sont purifiés dans les mêmes conditions que les composés de formule (I/a₁) :
les composés de formules (I/a₂) et (I/b₂) avant purification pouvant éventuellement être traités dans les mêmes conditions que les composés de formules (I/c₁), (I/c'₁) et (I/c"₁), pour conduire aux composés de formule (I/c₂), cas particulier des composés de formule (I) : 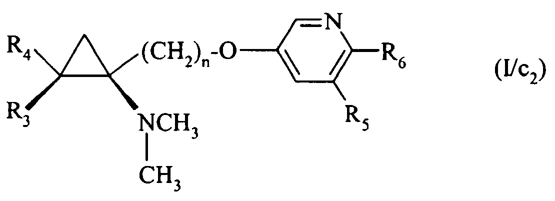
dans laquelle R₃, R₄, R₅, R₆ et n sont tels que définis précédemment, les composés de formule (I/c₂) sont purifiés dans les mêmes conditions que les composés de formule (I/a₁).

D'une façon générale, on comprend par isomères des composés de l'invention, les isomères optiques tels que les énantiomères et les diastéréoisomères. Plus particulièrement, les formes énantiomères pures des composés de l'invention peuvent être séparées à partir des mélanges d'énantiomères qui sont mis à réagir avec un agent libérable de dédoublement des racémiques, ledit agent existant quant à lui sous la forme d'un énantiomère pur, permettant d'obtenir les diastéréoisomères correspondants. Ces diastéréoisomères sont ensuite séparés selon des techniques de séparation bien connues de l'homme de l'art, telles que la cristallisation ou la chromatographie, puis l'agent de dédoublement est éliminé en utilisant les techniques classiques de la chimie organique, pour conduire à l'obtention d'un énantiomère pur.

Les composés de l'invention, qui sont présents sous la forme d'un mélange de diastéréoisomères, sont isolés sous forme pure par l'utilisation des techniques classiques de séparation telles que les chromatographies.

Dans certains cas particuliers, le procédé de préparation des composés de l'invention peut conduire à la formation prédominante d'un énantiomère ou d'un diastéréoisomère par rapport à l'autre.

Les composés de la présente invention, de part leurs propriétés pharmacologiques de ligands nicotiniques, et sélectivement du sous-type réceptoriel α4β2, sont utiles dans le traitement des déficits de mémoire associés au vieillissement cérébral et aux maladies neurodégénératives telles que la maladie d'Alzheimer, la maladie de Parkinson, la maladie de Pick, la maladie de Korsakoff et les démences frontales et sous-corticales, ainsi que pour le traitement des troubles de l'humeur, du syndrome de Tourette, du syndrome d'hyperactivité avec déficits attentionnels, du sevrage tabagique et de la douleur.

La présente invention a également pour objet les compositions pharmaceutiques renfermant comme principe actif au moins un composé de formule (I), un de ses isomères, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention.

Parmi les excipients ou véhicules inertes, non toxiques, pharmaceutiquement acceptables, on peut citer à titre indicatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants.

La posologie utile varie selon l'âge et le poids du patient, la voie d'administration, la composition pharmaceutique utilisées, la nature et la sévérité de l'affection, et la prise de traitements associés éventuels. La posologie s'échelonne de 1 mg à 500 mg en une ou plusieurs prises par jour.

Les exemples suivants illustrent l'invention.

Les produits de départ utilisés sont des produits connus ou préparés selon des modes opératoires connus. Les différentes préparations conduisent à des intermédiaires de synthèse utile pour la préparation des composés de l'invention.

Les structures des composés décrits dans les exemples et dans les préparations ont été déterminées selon les techniques spectrophotométriques usuelles (infrarouge, résonance magnétique nucléaire, spectrométrie de masse, ...).

Les points de fusion ont été déterminés soit à la platine chauffante de Kofler, soit à la platine chauffante sous microscope. Lorsque le composé existe sous forme de sel, le point de fusion donné ainsi que la microanalyse élémentaire correspondent à celui du produit salifié.

### PREPARATION 1 :

### (1S,2S), (1R,2R)-1-[(tert-Butoxycarbonyl)-amino]-2-méthylcyclopropane-carboxylate d'éthyle

### Stade 1 : 2-Méthyl-1,1-cyclopropanedicarboxylate de diéthyle

A une solution of 50,6 g d'iodure de trimethylsulfoxonium dans 400 cm³ de diméthyle sulfoxyde est ajouté en une seule fois 8.84 g d'hydrure de sodium à 60% dans l'huile. Le milieu réactionnel est agité 2 heures puis une solution de 43,3 g de diéthyl éthylidènemalonate dans 200 cm³ de diméthyle sulfoxyde est ajouté goutte à goutte en 15 minutes. Après 16 h d'agitation, un mélange de glace et de 100 cm³ d'acide chlorhydrique 1N est ajouté puis le milieu réactionnel est extrait à l'éther (3 x 200 cm³). Les phases organiques réunies sont lavées avec une solution de chlorure de sodium puis séchées sur sulfate de sodium et évaporées. Une chromatographie sur silice (dichlorométhane/ cyclohexane : 75/25) permet d'isoler 37,5 g de produit attendu.

### Stade 2 : Acide (1R,2S),(1S,2R)-1-(éthoxycarbonyl)-2-méthylcyclopanecarboxylique

195 cm³ d'une solution d'hydroxyde de sodium 1N sont ajoutés goutte à goutte à une solution de 37,5 g du composé obtenu au stade 1 précédent dans 400 cm³ d'éthanol La réaction est agitée 16 heures à température ambiante. Après évaporation de l'éthanol et dilution avec 50 cm³ d'une solution aqueuse de chlorure de sodium, la phase aqueuse résiduelle est lavée à l'éther (2 x 50 cm³) puis acidifiée avec 48 cm³ d'acide chlorhydrique 4N. Le milieu réactionnel est de nouveau extrait à l'éther (2 x 100 cm³), les phases organiques réunies sont séchées sur sulfate de sodium et évaporées pour obtenir 29,4 g du produit attendu.
Rapport diastéréoisomérique : 95/5

### Stade 3 : (1S,2S), (1R,2R)-1-[(tert-Butoxycarbonyl)amino]-2-méthylcyclopropanecarboxylate d'éthyle

Sous atmosphère inerte et à température ambiante, une solution de 22 cm³ de diphénylphosphorylazide dans 30 cm³ de toluène est additionnée goutte à goutte, à une solution de 17,2 g du composé obtenu au stade 2 précédent, de 13 cm³ de triéthylamine, et de 27 cm³ de *tert*-butanol dans 150 cm³ de toluène. Le milieu réactionnel est ensuite chauffé 16 heures à 85 °C sous agitation. Le milieu réactionnel refroidi est lavé avec une solution de carbonate de sodium puis avec une solution de chlorure de sodium. Après séchage sur sulfate de sodium, évaporation et chromatographie sur silice (dichlorométhane/cyclohexane : 75/25 puis dichlorométhane/tétrahydrofuranne : 98/2), 14,03 g du produit attendu sont obtenus.

### PREPARATION 2 :

### tert-butyl (1S,2S),(1R,2R)-1-(Hydroxyméthyl)-2-méthylcyclopropylcarbamate

41 cm³ d'une solution de borohydrure de lithium 2M dans le tétrahydrofuranne sont additionnés goutte à goutte à une solution de 10 g du produit de la préparation 1 dans 40 cm³ de tétrahydrofuranne. La solution est agitée 20 heures à température ambiante. Après refroidissement dans un bain de glace et hydrolyse avec 20 cm³ d'eau, le milieu réactionnel est séché sur sulfate de sodium puis le tétrahydrofuranne est évaporé. La chromatographie sur silice du résidu obtenu (dichlorométhane puis dichlorométhane/méthanol : 95/5), permet d'obtenir 7,2 g du produit attendu.

### PREPARATION 3 :

### [(1R,2S), (1S,2R)-2-Méthyl-1-(méthylamino)cyclopropyl]méthanol

### Stade 1 : (1R,2S), (1S,2R)-1-Isocyano-2-méthylcyclopropanecarboxylate d'éthyle

Une solution de 2,5 g d'isocyanate d'éthyle, 2,3 cm³ de 1,2-dibromopropane, 25 cm³ de diméthyle sulfoxyde et 60 cm³ d'éther est additionnée goutte à goutte, en une heure, à une suspension de 1,93 g d'hydrure de sodium à 60 % dans l'huile dans 20 cm³ d'éther. Après 2 heures de chauffage au reflux, le milieu réactionnel est refroidi et coulé sur un mélange de 50 cm³ d'eau glacée et 50 cm³ d'éther. La phase aqueuse est décantée et extraite de nouveau à l'éther (3 x 40 cm³). Les phases organiques réunies sont lavées avec une solution aqueuse de chlorure de sodium, séchées sur sulfate de sodium et évaporées. La chromatographie sur silice (dichlorométhane/tétrahydrofuranne : 97/3), permet d'obtenir 4,88 g de produit attendu.
Rapport diastéréoisomérique : 90/10.

### Stade 2 : [(1R,25), (1S,2R)-2-Méthyl-1-(méthylamino)cyclopropyl]méthanol

Une solution de 4,88 g du composé obtenu au stade 1 précédent dans 85 cm³ d'éther est additionnée goutte à goutte à une suspension de 3,73 g d'hydrure d'aluminium lithium dans 250 cm³ d'éther. Le mélange réactionnel est porté 4 heures au reflux puis agité 16 heures à température ambiante. Le milieu réactionnel est refroidi dans un bain de glace avant ajout de sulfate de sodium imprégné d'eau. Après deux heures d'agitation les minéraux sont filtrés, la phase éthérée est séchée sur sulfate de sodium puis évaporée pour obtenir 2,75 g du produit attendu.
Rapport diastéréoisomérique : 90/10.

### Exemple 1 :

### Chlorhydrate de (1S,2S), (1R,2R)-N,2-diméthyl-1-[(3-pyridinyloxy)méthyl]cyclopropanamine

### Stade 1 : (1S,2S), (1R,2R)-1-[Benzyl(tert-butoxycarbonyl)amino]-2-méthylcyclopropanecarboxylate d'éthyle

21,5 g de *tert*-butylate de potassium sont ajoutés à température ambiante, à une solution de 39 g du composé de la préparation 1 dans 400 cm³ de diméthylformamide. Le milieu réactionnel est agité une heure puis 35,9 g de bromure de benzyle sont additionnés en 30 minutes. Après 16 heures d'agitation, le diméthylformamide est évaporé. Le résidu est repris par 400 cm³ d'éther. La phase organique obtenue est lavée successivement avec des solutions de chlorure de lithium et de carbonate de sodium puis séchée sur sulfate de sodium et évaporée. La chromatographie sur silice (dichlorométhane puis dichlorométhane/butanone : 95/5), permet d'obtenir 41,4 g de produit attendu.

### Stade 2 : (1S,2S), (1R,2R)-1-[Benzyl(méthyl)amino]-2-méthylcyclopropanecarboxylate d'éthyle

Une solution de 41,2 g du composé obtenu au stade 1 précédent dans 410 cm³ d'acide formique et 410 cm³ d'une solution aqueuse à 37 % de formaldéhyde est portée deux heures trente au reflux. Le milieu réactionnel est ensuite concentré et repris par un mélange éther/solution aqueuse de carbonate de sodium. Après filtration d'un insoluble, la phase aqueuse est décantée puis de nouveau extraite à l'éther. Les phases éthérées réunies sont séchées sur sulfate de sodium puis concentrées. Le résidu est repris par 200 cm³ de cyclohexane. On obtient, après filtration d'un léger insoluble et évaporation du cyclohexane, 30 g du produit attendu.

### Stade 3: {(1S,2S), (1R,2R)-1-[Benzyl(méthyl)amino]-2-méthylcyclopropyl}méthanol

Une solution de 29,7 g du composé obtenu au stade 2 précédent dans 150 cm³ d'éther est additionnée goutte à goutte à une suspension de 4,55 g d'hydrure d'aluminium lithium dans 300 cm³ d'éther. Le mélange réactionnel est agité 20 heures puis refroidi dans un bain de glace avant ajout de sulfate de sodium imprégné d'eau. Après deux heures d'agitation les minéraux sont filtrés, la phase éthérée est séchée sur sulfate de sodium puis évaporée pour donner 24 g du produit attendu.

### Stade 4 : (1S,2S), (1R,2R)-N-Benzyl-N,2-diméthyl-1-[(3-pyridinyloxy)méthyl]cyclopropanamine

Dans un réacteur de 80 cm³, 2,4 g de *tert*-butylate de potassium sont ajoutés, à une solution de 4 g du composé obtenu au stade 3 précédent et de 6 cm³ de 3-fluoropyridine dans 40 ml de diméthyle sulfoxyde. Le milieu réactionnel est chauffé 8 minutes à 120°C dans un four à micro-ondes monomode. La totalité de l'opération est répétée deux fois. Les trois essais réunis sont coulés sur 600 cm³ d'une aqueuse solution de carbonate de sodium. Le milieu réactionnel est extrait trois fois à l'éther. Les phases éthérées réunies sont lavées avec une solution de carbonate de sodium, séchées sur sulfate de sodium et concentrées pour donner 16,3 g de produit attendu.

### Stade 5 : Chlorhydrate de (1S,2S), (1R,2R)-N,2-diméthyl-1-[(3-pyridinyloxy)méthyl]-cyclopropanamine

28,4 cm³ d'acide chlorhydrique 1 N sont ajoutés à une solution de 4 g du composé obtenu au stade 4 précédent dans 120 cm³ d'éthanol. Le milieu réactionnel est hydrogéné 2 heures sous pression atmosphérique à température ambiante en présence de 1 g d'hydroxyde de palladium (20% sur charbon). Le catalyseur est filtré et les solvants évaporés. Le résidu est repris dans l'éthanol et concentré de nouveau pour donner 3,6 g du produit attendu.
Spectrométrie de masse (ESI) : m/z = 193,1 Th [M+H]⁺
Point de fusion (cap) : 188-190°C (recristallisation dans l'éthanol)

### Stade 6 : Séparation des énantiomères du composé de l'exemple 1

3,5 g de composé de l'exemple 1 sont chromatographiés sur colonne Chiralpack AD, (Ethanol, diéthylamine), pour obtenir respectivement, après salification par une solution éthanolique d'acide chlorhydrique et cristallisation, 2 g du dichlorhydrate d'un premier énantiomère α et 2,1 g du dichlorhydrate du second énantiomère β.

### Exemple 2 (énantiomère α) :

### Dichlorhydrate de (1S,2S) ou (1R,2R)-(-)-N,2-diméthyl-1-[(3-pyridinyloxy)méthyl ]cyclopropanamine

Spectrométrie de masse (EI) : m/z = 192 Th (M⁺)
Point de fusion (cap) : 174-176°C
Pouvoir rotatoire : [α]_{D}^{19,7} : -19,46° (c=0,01 g/cm³, Ethanol).

### Exemple 3 (énantiomère β) :

### Dichlorhydrate de (1S,2S) ou (1R,2R)-(+)-N,2-diméthyl-1-[(3-pyridinyloxy)méthyl]-cyclopropanamine

Spectrométrie de masse (CI) (NH₃) : m/z = 193 Th ([M+H]⁺)
Point de fusion (cap) : 174-176°C
Pouvoir rotatoire : [α]_{D}^{19,7} : + 17° (c=0,01 g/cm³, Ethanol)

### Exemple 4 :

### (1S,2S), (1R,2R)-2-Méthyl-1-[(3-pyridinyloxy)méthyl]cyclopropanamine

### Stade 1 : Chlorhydrate de [(1S,2S), (1R,2R)-1-amino-2-méthylcyclopropyl]methanol

20 cm³ d'une solution d'acide chlorhydrique 4N dans le dioxanne sont ajoutés à une solution de 7 g du composé de la préparation 2 dans 20 cm³ d'éthanol. Après 20 h d'agitation, les solvants sont évaporés. 20 cm³ de dioxanne et 150 cm³ d'éther sont ajoutés au résidu et après 20 heures d'agitation, le chlorhydrate attendu est filtré et séché. 4,6 g du produit attendu sont obtenus.

### Stade 2 : (1S,2S), (1R,2R)-2-Méthyl-1-[(3-pyridinyloxy)méthyl]cyclopropanamine

12,5 g de *tert*-butylate de potassium sont ajoutés à une solution de 4,5 g du produit obtenu au stade 1 précédent dans 75 cm³ de diméthyle sulfoxyde. Après 30 minutes d'agitation, 22 cm³ de 3-fluoropyridine sont ajoutés et l'agitation est poursuivie 6 heures. Le milieu réactionnel est coulé sur une solution aqueuse de carbonate de sodium et extrait avec de l'acétate d'éthyle. La phase organique est lavée avec une solution aqueuse de carbonate de sodium puis séchée sur sulfate de sodium et concentrée. La chromatographie sur silice du résidu obtenu (dichlorométhane/méthanol : 95/5), permet d'obtenir 3,2 g de produit attendu.

### Stade 3 : Séparation des énantiomères du composé de l'exemple 4

3,15 g du composé de l'exemple 4 sont chromatographiés sur colonne Chiralpack AD (méthanol, diéthylamine), pour obtenir respectivement, après salification par une solution d'acide chlorhydrique 4N dans le dioxanne et cristallisation, 1,75 g du dichlorhydrate d'un premier énantiomère α et 1,7 g du dichlorhydrate du second énantiomère β.

### Exemple 5 (énantiomère α) :

### Dichlorhydrate de (1S,2S) ou (1R,2R)-(-)-2-méthyl-1-(3-pyridinyl-oxy)méthyl]cyclopropanamine

Spectrométrie de masse (ESI) : m/z = 179,1 Th ([M+H]⁺)
Point de fusion : < 75°C (gomme)
Pouvoir rotatoire : [α]_{D}²⁰ : -23,14° (c=0,01054 g/cm³, Ethanol).

### Exemple 6 (énantiomère β) :

### Dichlorhydrate de (1S,2S) ou (1R,2R)-(+)-2-méthyl-1-(3-pyridinyl-oxy)méthyl]cyclopropanamine

Spectrométrie de masse (ESI) : m/z = 179,1 Th ([M+H]⁺)
Point de fusion : < 75°C (gomme)
Pouvoir rotatoire : [α]_{D}²⁰: = +22,14° (c=0,01055 g/cm³, Ethanol)

### Exemple 7 :

### (1S,2S), (1R,2R)-N,N,2-Triméthyl-1-[(3-pyridinyloxy)méthyl]cyclopropanamine

### Stade 1: (1S,2S), (1R,2R)-N,N,2-Triméthyl-1-[(3-pyridinyloxy)méthyl]cyclopropanamine

Une solution de 3,6 g du composé de l'exemple 4 dans 40 cm³ d'acide formique et 40 cm³ d'une solution aqueuse à 37 % de formaldéhyde est portée 4 heures au reflux. Le milieu réactionnel est coulé sur une solution aqueuse de carbonate de sodium et extrait à l'acétate d'éthyle. La phase organique est séchée sur sulfate de sodium puis concentrée. La chromatographie sur silice du résidu obtenu (dichlorométhane/méthanol : 97/3), permet d'obtenir 2,6 g du composé attendu.

### Stade 2 : Séparation des énantiomères du composé de l'exemple 7

3,9 g du composé de l'exemple 7 sont chromatographiés sur colonne Chiralpack AD (méthanol, acétonitrile, diéthylamine), pour obtenir respectivement, après salification par une solution d'acide chlorhydrique 4N dans le dioxanne, 2,3 g du dichlorhydrate du premier énantiomère α et 2,3 g du dichlorhydrate du second énantiomère β.

### Exemple 8 (énantiomère α) :

### Dichlorhydrate de (1S,2S), (1R,2R)-(+)-N,N,2-triméthyl-1-[(3-pyridinyloxy)méthyl]-cyclopropanamine

Spectrométrie de masse (ESI) : m/z = 201,7 Th ([M+H]⁺)
Point de fusion (cap) : 171-173°C
Pouvoir rotatoire : [α]_{D}²⁰: = +15,79° (c=0,01014 g/cm³, Ethanol)

### Exemple 9 (énantiomère β) :

### Dichlorhydrate de (1S,2S), (1R,2R)-(-)-N,N,2-triméthyl-1-[(3-pyridinyloxy)méthyl]-cyclopropanamine

Spectrométrie de masse (ESI) : m/z = 207,1 Th ([M+H]⁺)
Point de fusion (cap) : 172-173°C
Pouvoir rotatoire : [α]_{D}²⁰ : = -16,64° (c=0,01016 g/cm³, Ethanol)

### Exemple 10 :

### (1S,2S), (1R,2R)-1-{[(6-Chloro-3-pyridinyl)oxy]méthyl}-N,2-diméthylcyclopropanamine

### Stade 1 : (1S,2S), (1R,2R)-1-[(tert-Butoxycarbonyl)(méthyl)amino]-2-méthylcyclopropane-carboxylate d'éthyle

5,5g de *tert*-butylate de potassium sont ajoutés à température ambiante, à une solution de 11,3 g du composé de la préparation 1 dans 175 cm³ de diméthylformamide. Le milieu réactionnel est agité 45 minutes puis 3,2 cm³ d'iodure de méthyle sont additionnés goutte à goutte. Après 16 heures d'agitation, 200 cm³ d'une solution aqueuse de carbonate de sodium sont ajoutés et le milieu réactionnel est extrait trois fois à l'éther. Les phases organiques réunies sont lavées avec une solution de chlorure de lithium puis séchées sur sulfate de sodium et évaporées. La chromatographie sur silice (dichlorométhane/cyclohexane : 75/25), permet d'obtenir 9,17 g du produit attendu.

### Stade 2 : tert-butyl (1S,2S), (1R,2R)-1-(Hydroxyméthyl)-2-méthylcyclopropyl (méthyl)carbamate

65 cm³ d'une solution de borohydrure de lithium 2M dans le tétrahydrofuranne sont additionnés goutte à goutte à une solution de 10 g du composé obtenu au stade 1 précédent dans 45 cm³ de tétrahydrofuranne. La solution est agité trois jours à 60°C. Après refroidissement dans un bain de glace et hydrolyse avec 100 cm³ d'eau, le milieu réactionnel est extrait trois fois à l'éther. Les phases organiques réunies sont séchées sur sulfate de sodium et évaporées. La chromatographie sur silice (dichlorométhane/tétrahydrofuranne : 97/3), permet d'obtenir 5,26 g du produit attendu.

### Stade 3 : tert-butyl (1S,2S), (1R,2R)-1-(Bromométhyl)-2-méthylcyclopropyl (méthyl)carbamate

2,42 g de triphénylphosphine puis 3,05 g de tétrabromométhane sont successivement ajoutés à une solution 2 g du composé obtenu au stade 2 précédent dans 48 cm³ d'éther.

Après 20 heures d'agitation le milieu réactionnel est filtré et le filtrat est concentré. La chromatographie sur silice (dichlorométhane/cyclohexane : 75/25), permet d'obtenir 1,85 g du produit attendu.

### Stade 4 : tert-butyl (1S,2S), (1R,2R)-1-{[(6-Chloro-3-pyridinyl)oxy]méthyl}-2-méthylcyclopropyl-(méthyl)carbamate

12,4 g de carbonate de césium sont ajoutés à une solution de 5,3 g du composé obtenu au stade 3 précédent et de 4,9 g de 6-chloropyridin-3-ol dans 100 cm³ de butanone. Après 20 heures de reflux, les minéraux sont filtrés et la butanone évaporée. Le résidu est repris par l'acétate d'éthyle, la phase organique obtenue est lavée avec une solution aqueuse de carbonate de sodium puis séchée sur sulfate de sodium et concentrée. La chromatographie sur silice (dichlorométhane/butanone : 95/5), permet d'obtenir 4 g du produit attendu.

### Stade 5 : (1S,2S), (1R,2R)-1-{[(6-Chloro-3-pyridinyl)oxy]méthyl}-N,2-diméthylcyclopropanamine

15 cm³ d'une solution d'acide chlorhydrique 4N dans le dioxanne sont ajoutés à une solution de 4 g du composé obtenu au stade 4 précédent dans 15 cm³ de dioxanne. Après 20 heures d'agitation, le milieu réactionnel est dilué à l'éther puis 3,5 g de chlorhydrate du produit attendu sont filtrés. Le chlorhydrate est repris par une solution aqueuse de carbonate de sodium. Après extraction par le dichlorométhane, séchage de la phase organique sur sulfate de sodium et évaporation du solvant, on obtient 2,5 g du produit attendu.

### Stade 6 : Séparation des énantiomères du composé de l'exemple 10

4,5 g du composé de l'exemple 10 sont chromatographiés sur colonne Chiralpack AD (méthanol/diéthylamine : 1000/1), pour obtenir respectivement, après salification par une solution d'acide chlorhydrique 4N dans le dioxanne et cristallisation, 2,35 g du dichlorhydrate du premier énantiomère α et 2,2 g du dichlorhydrate du second énantiomère β.

### Exemple 11 (énantiomère α) :

### Chlorhydrate de (1S,2S) ou (1R,2R)-(-)-1-{[(6-chloro-3-pyridinylroxy]méthyl}-N,2-diméthylcyclopropanamine

Spectrométrie de masse (ESI) : m/z = 227,1 Th ([M+H]⁺)
Point de fusion (cap) : 185-190°C
Pouvoir rotatoire : [α]_{D}²⁰: = -13,90° (c=0,01079 g/cm³, Méthanol)

### Exemple 12 (énantiomère β) :

### Chlorhydrate de (1S,2S) ou (1R,2R)-(+)-1-{[(6-chloro-3-pyridinyl)-oxy]méthyl}-N,2-diméthylcyclopropanamine

Spectrométrie de masse (ESI) : m/z = 227,1 Th ([M+H]⁺)
Point de fusion (cap) : 188-192°C
Pouvoir rotatoire : [α]_{D}²⁰: = +15,13° (c=0,01062 g/cm³, Méthanol)

### Exemple 13 :

### (1S,2S), (1R,2R)-1-{[(6-Chloro-3-pyridinyl)oxy]méthyl}-2-méthylcyclopropanamine

### Stade 1 : tert-butyl (1S,2S), (1R,2R)-1-(Bromométhyl)-2-méthylcyclopropylcarbamate

Le composé est obtenu selon le procédé du stade 3 de l'exemple 10 en remplaçant le composé du stade 2 de l'exemple 10 par le composé de la préparation 2.

### Stade 2 : tert-butyl (1S,2S), (1R,2R)-1-{[(6-Chloro-3-pyridinyl)oxy]méthyl}-2-méthylcyclopropyl-carbamate

Le composé est obtenu selon le procédé du stade 4 de l'exemple 10 en remplaçant le composé du stade 3 de l'exemple 10 par le composé du stade 1 précédent.

### Stade 3 : (1S,2S), (1R,2R)-1-{[(6-Chloro-3-pyridinyl)oxy]méthyl}-2-méthylcyclopropanamine

Le composé est obtenu selon le procédé du stade 5 de l'exemple 10 en remplaçant le composé du stade 4 de l'exemple 10 par le composé du stade 2 précédent.

### Stade 4 : Séparation des énantiomères du composé de l'exemple 13

2 g du composé de l'exemple 13 sont chromatographiés sur colonne Chiralpack AD (isopropanol/diéthylamine), pour obtenir respectivement après salification par une solution d'acide chlorhydrique 4N dans le dioxanne et cristallisation, 1,05 g du chlorhydrate du premier énantiomère α et 1,1 g du chlorhydrate du second énantiomère β.

### Exemple 14 (énantiomère α) :

### Chlorhydrate de (1S,2S) ou (1R,2R)-(-)-1-{[(6-chloro-3-pyridinyl)-oxy]méthyl}-2-méthylcyclopropanamine

Spectrométrie de masse (ESI) : m/z = 213,1 Th ([M+H]⁺)
Point de fusion (cap) : 183-186°C
Pouvoir rotatoire : [α]_{D}²⁰: = -15,89° (c=0,0101 g/cm³, Méthanol)

### Exemple 15 (énantiomère β) :

### Chlorhydrate de (1S,2S) ou (1R,2R)-(+)-1-{[(6-chloro-3-pyridinyl)-oxy]méthyl}-2-méthylcyclopropanamine

Spectrométrie de masse (ESI) : m/z = 213,1 Th ([M+H]⁺)
Point de fusion (cap) : 183-186°C
Pouvoir rotatoire : [α]_{D}²⁰: = +16,49° (c=0,01015 g/cm³, Méthanol)

### Exemple 16 :

### (1S,2S), (1R,2R)-1-}[(6-Chloro-3-pyridinyl)oxy]méthyl}-N,N,2-triméthylcyclopropanamine

### Stade 1: (1S,2S), (1R,2R)-1-{[(6-Chloro-3-pyridinyl)oxy]méthyl}-N,N,2-triméthylcyclopropanamine

Le composé est obtenu selon le procédé du stade 1 de l'exemple 7 en remplaçant le composé de l'exemple 4 par le composé de l'exemple 10.

### Stade 2 : Séparation des énantiomères du composé de l'exemple 16

2,4 g du composé de l'exemple 16 sont chromatographiés sur colonne Chiralpack AD (isopropanol/diéthylamine : 100/1), pour obtenir respectivement , après salification par une solution d'acide chlorhydrique 4N dans le dioxanne et cristallisation, 1,1 g du chlorhydrate du premier énantiomère α et 1,05 g du chlorhydrate du second énantiomère β.

### Exemple 17 (énantiomère α) :

### Chlorhydrate de (1S,2S) ou (1R,2R)-(+)-1-{[(6-chloro-3-pyridinyl)-oxy]méthyl}-N,N,2-triméthylcyclopropanamine

Spectrométrie de masse (ESI) : m/z = 241,1 Th ([M+H]⁺)
Point de fusion (cap) : 136-138°C
Pouvoir rotatoire : [α]_{D}²⁰: = +12,64° (c=0,01055 g/cm³, Méthanol)

### Exemple 18 (énantiomére α) :

### Chlorhydrate de (1S,2S) ou (1R,2R)-(-)-1-{[(6-chloro-3-pyridinyl)-oxy]méthyl]-N,N,2-triméthylcyclopropanamine

Spectrométrie de masse (ESI) : m/z = 241,1 Th ([M+H]⁺)
Point de fusion (cap) : 136-138°C
Pouvoir rotatoire : [α]_{D}²⁰ := -11,23° (c=0,01055 g/cm³, Méthanol)

### Exemple 19 :

### Dichlorhydrate de (1S,2R), (1R,2S)-N,2-Diméthyl-1-[(3-pyridinyloxy)méthyl]-cyclopropanamine

### Stade 1 : Dichlorhydrate de (1S,2R), (1R,2S)-N,2-Diméthyl-1-[(3-pyridinyloxy)méthyl] cyclopro-panamine

2,6 g de *tert*-butylate de potassium et 9,2 cm³ de 3-fluoropyridine sont ajoutés à une solution de 2,25 g du composé de la préparation 3 dans 75 cm3 de diméthyle sulfoxyde. Le milieu réactionnel est chauffé 90 secondes à 30% de la puissance maximum d'un four à micro-ondes multimode de 1000 W. Après refroidissement, le milieu réactionnel est coulé sur une solution aqueuse de chlorure de sodium et extrait plusieurs fois à l'acétate d'éthyle. Les phases organiques réunies sont lavées avec une solution de chlorure de sodium puis séchées sur sulfate de sodium et évaporées. La chromatographie sur silice, (dichlorométhane/méthanol/ammoniaque), permet d'obtenir 2,46 g du produit non salifié sous forme d'un diastéréoisomère unique. Après salification du composé par une solution d'acide chlorhydrique 4N dans le dioxanne et cristallisation, on obtient 1,22 g du composé attendu.
Spectrométrie de masse (ESI) : m/z = 193,1 Th ([M+H]⁺)
Point de fusion (cap) : 189-191°C

### Stade 2 : Séparation des énantiomères du composé de l'exemple 19

1,8 g du composé de l'exemple 19 sont chromatographiés sur colonne Chiralpack AD (ethanol/acétonitrile/diéthylamine: 150/850/1), pour obtenir respectivement, après salification par une solution d'acide chlorhydrique 4N dans le dioxanne et cristallisation, 0,54 g du chlorhydrate du premier énantiomère α et 0 58 g du chlorhydrate du second énantiomère β.

### Exemple 20 (énantiomère α) :

### Dichlorhydrate de (1S,2R) ou (1R,2S)-(+)-N,2-diméthyl-1-[(3-pyridinyloxy)méthyl] cyclopropanamine

Spectrométrie de masse (ESI) : m/z = 193,1 Th ([M+H]⁺)
Point de fusion (cap) : 69-72°C
Pouvoir rotatoire : [α]_{D}²⁰ : = +30,04° (c=0,01084 g/cm³, Ethanol)

### Exemple 21 (énantiomère α) :

### Dichlorhydrate de (1S,2R) ou (1R,2S)-(-)-N,2-diméthyl-1-[(3-pyridinyloxy)méthyl] cyclopropanamine

Spectrométrie de masse (ESI) : m/z = 193,1 Th ([M+H]⁺)
Point de fusion (cap) : 71-74°C
Pouvoir rotatoire : [α]_{D}²⁰ : = -31,24° (c=0,00961 g/cm³, Ethanol)

### Exemple 22 :

### Dichlorhydrate de (1S,2R), (1R,2S)-1-{[(5-bromo-3-pyridinyl)oxy]méthyl}-N,2-diméthylcyclopropan amine

1,7 g d'hydrure de sodium à 60% dans l'huile sont ajoutés à 4,6 g du composé de la préparation 3 dans 160 cm³ de diméthylformamide. Le milieu réactionnel est agité une heure à température ambiante puis 10,2 g de 3,5-dibromopyridine sont additionnés goutte à goutte. Le milieu réactionnel est chauffé 16 heures à 60°C puis le diméthylformamide est évaporé. Le résidu est repris dans 300 cm³ d'éther. La phase organique est lavée avec une solution aqueuse de chlorure de lithium puis séchée sur sulfate de sodium et concentrée. La chromatographie sur silice (dichlorométhane/méthanol : 96/4), permet d'obtenir 4,86 g du composé non salifié. Après salification de 0,43 g du composé dans l'éthanol par 1 cm³ d'une solution d'acide chlorhydrique 4N dans le dioxanne, les solvants sont évaporés et le résidu est recristallisé dans l'éthanol pour obtenir 0,35g du produit attendu.
Spectrométrie de masse (ESI) : m/z = 271,1 Th ([M+H]⁺)
Point de fusion (cap) : 157-159°C

### Exemple 23 :

### Dichlorhydrate de (1S,2R), (1R,2S)-2-méthyl-1-[(3-pyridinyloxy)méthyl]cyclopropanamine

### Stade 1 : (2E)-2-Cyano-2-butènoate d'éthyle

2 cm³ de pipéridine sont ajoutés goutte à goutte à une solution de 64 cm³ d'ethyl cyanoacétate et de 34 cm³ d'acétaldéhyde dans 80 cm³ d'acide acétique. Après 20 heures d'agitation à température ambiante, le milieu réactionnel est dilué avec 200 cm³ d'eau et extrait à l'éther (3 x 200 cm³). Les phases organiques réunies sont lavées à l'eau puis séchées sur sulfate de sodium et évaporées. La distillation sous pression réduite (97°C/10 torr) du résidu obtenu permet d'obtenir 40,44 g du produit attendu.
Rapport diastéréoisomérique E/Z : 96/4.

### Stade 2 : (1S,2S), (1R,2R)-1-Cyano-2-méthylcyclopropanecarboxylate d'éthyle

A une solution of 17,6 g d'iodure de triméthylsulfoxonium dans 120 cm³ de diméthyle sulfoxyde est ajouté en une seule fois 1,92 g d'hydrure de sodium à 60% dans l'huile. Le milieu réactionnel est agité 2 heures puis une solution de 10 g du composé obtenu au stade 1 précédent dans 60 cm³ de diméthyle sulfoxyde est ajoutée goutte à goutte. Après 16 heures d'agitation, le milieu réactionnel est coulé sur un mélange de glace et d'acide chorhydique 1N puis est extrait à l'éther (3 x 200 cm³). Les phases organiques réunies sont lavées avec une solution aqueuse de chlorure de sodium puis séchées sur sulfate de sodium et évaporées. Une chromatographie sur silice (dichlorométhane/tétrahydrofuranne : 97/3), permet d'isoler 7,15 g du produit attendu.

### Stade 3 : (1S,2S), (1R,2R)-1-(Hydroxyméthyl)-2-méthylcyclopropanecarbonitrile

Une solution de 2 g du composé obtenu au stade 2 précédent dans 20 cm³ de tétrahydrofuranne est ajouté goutte à goutte à une solution de 4,92 g de borohydrure de sodium dans 80 cm³ de tétrahydrofuranne et 5 cm³ d'eau. La solution est ensuite agitée 7 heures à 50°C puis la nuit à température ambiante. 100 cm³ de méthanol sont additionnés avec précaution puis le milieu réactionnel est concentré. L'opération est répétée une fois puis le résidu est repris par 100 cm³ d'hydrogénocarbonate de sodium et extrait au dichlorométhane. Après une seconde extraction au dichlorométhane, les phases organiques réunies sont séchées sur sulfate de sodium et évaporées. La chromatographie sur silice (dichlorométhane/tétrahydofuranne : 97/3) permet d'obtenir 1,71 g du produit attendu.

### Stade 4 : (1S,2S), (1R,2R)-2-Méthyl-1-[(3-pyridinyloxy)méthyl]cyclopropanecarbonitrile

2,4 g de *tert*-butylate de potassium sont ajoutés à une solution de 0,56 g du produit du stade 3 précédent dans 15 cm³ de diméthyle sulfoxyde. Après homogénéisation, 2,2 cm³ de 3-fluoropyridine sont ajoutés et la réaction est chauffée 5 minutes à 80°C. Après refroidissement, le milieu réactionnel est dilué avec 40 cm³ d'une solution aqueuse de chlorure de sodium et extrait plusieurs fois à l'acétate d'éthyle. Les phases organiques réunies sont lavées avec une solution de chlorure de sodium puis séchées sur sulfate de sodium et évaporées. La chromatographie sur silice (dichlorométhane/tétrahydrofuranne : 97/3) permet d'obtenir 0,71 g du produit attendu.

### Stade 5 : (1R,2S), (1S,2R)-2-Méthyl-1-[(3-pyridinyloxy)méthyl]cyclopropanecarboxamide

Un mélange de 1 g du produit obtenu au stade 4 précédent, 3,8 cm³ d'une solution aqueuse à 50 % d'eau oxygénée et 0,32 g d'hydroxyde de lithium dans 10 cm³ d'éthanol est agité 45 minutes à température ambiante puis 75 minutes à 45°C. Le milieu réactionnel est filtré et les insolubles sont lavés au dichlorométhane. Le filtrat est concentré et la chromatographie sur silice, (dichlorométhane/méthanol/ammoniaque : 95/4/1) permet d'obtenir 0,94 g du produit attendu.

### Stade 6 : Dichlorhydrate de (1S,2R), (1R,2S)-2-méthyl-1-[(3-pyridinyloxy)méthyl]-cyclopropanamine

0,25 cm³ de brome sont ajoutés à une solution de 1,64 g d'hydroxyde de potassium dans 10 cm³ d'eau. La solution est refroidie à 0°C et 1 g du produit préparé au stade 5 précédent est ajouté en une fois. Le milieu réactionnel est agité vigoureusement 15 minutes à température ambiante puis 45 minutes à 75°C. Après refroidissement, le milieu réactionnel est dilué avec 30 cm³ d'une solution aqueuse de chlorure de sodium et extrait plusieurs fois à l'acétate d'éthyle. Les phases organiques réunies sont séchées sur sulfate de sodium et évaporées. La chromatographie sur silice (dichlorométhane/méthanol/ ammoniaque : 95/4/1) permet d'obtenir 0,59 g du produit non salifié. Après salification de 1,18 g du composé par une solution d'acide chlorhydrique 4N dans le dioxanne et recristallisation dans l'éthanol, 1,41 g du produit attendu sont obtenus.
Spectrométrie de masse (ESI) : m/z = 179 Th ([M+H]⁺)
Point de fusion (cap) : 188-190°C

### Exemple 24 :

### Dichlorhydrate de (1S,2R), (1R,2S)-N,N-2-triméthyl-1-[(3-pyridinyloxy)méthyl]cyclopropanamine

Le composé est obtenu selon le procédé du stade 1 de l'exemple 7 en remplaçant le composé de l'exemple 4 par le composé de l'exemple 23. Après salification de 0,88 g du composé par une solution d'acide chlorhydrique 4N dans le dioxanne, les solvants sont évaporés et le résidu est recristallisé dans l'éthanol pour obtenir 0,98 g du produit attendu.
Spectrométrie de masse (ESI) : m/z = 207,1 Th ([M+H]⁺)
Point de fusion (cap) : 196-200°C

### Exemple 25 :

### Dichlorhydrate de (R,S)-N,2,2-triméthyl-1-[(3-pyridinyloxy)méthyl]cyclopropanamine

### Stade 1 : 2-(2-Méthylpyropylidène)malonate de diéthyle

Un mélange de 69,80 g de 2-méthyl-propanal, 77,30 g de diéthylmalonate, 2,06 g d'acide benzoïque, 2,47 cm³ de pipéridine et 100 cm³ de toluène est agité à reflux pendant 15 heures. Après refroidissement, 100 cm³ de toluène sont ajoutés. La solution est lavée successivement 2 fois avec 100 cm³ d'eau distillée, 2 fois avec 100 cm³ d'acide chlorhydrique 1M et 100 cm³ de solution aqueuse saturée de carbonate de sodium. La phase organique est séchée sur sulfate de sodium anhydre et concentrée. Le résidu obtenu est distillé sous pression réduite (117°C/10 torr) pour obtenir 93,44 g de produit attendu sous forme d'une huile incolore.

### Stade 2 : 2-(2-Bromo-2-méthylpropylidène)malonate de diéthyle

56,04 g de *N*-bromosuccinimide sont ajoutés à une solution de 64,20 g du composé obtenu au stade 1 précédent dans 300 cm³ de tétrachlorure de carbone. Le milieu réactionnel est agité à reflux pendant 2 heures. Après refroidissement à température ambiante, le mélange est filtré et concentré pour obtenir 89,8 g de produit attendu.

### Stade 3 : 2,2-Diméthyl-1,1-cyclopropanedicarboxylate de diéthyle

Une solution de 87,70 g du composé obtenu au stade 2 précédent dans 90 cm³ d'éthanol est additionnée goutte à goutte à 24,3 g de borohydrure de sodium dans 300 cm³ d'éthanol. Le milieu réactionnel est agité trois heures à température ambiante, puis chauffé à 40°C pendant 1h30. Le solvant est évaporé, le résidu est repris par 200 cm³ d'eau distillée et extrait au dichlorométhane (3 x 200 cm³⁾. Les phases organiques réunies sont séchées sur sulfate de sodium et concentrées pour obtenir 63,0 g de produit attendu.

### Stade 4 : Acide 1-(Ethoxycarbonyl)-2,2-diméthylcyclopropanecarboxylique

270 cm³ d'hydroxyde de sodium 1 N sont ajoutés à température ambiante, en 30 minutes, à une solution de 59,2 g du composé obtenu au stade 3 précédent dans 600 cm³ d'éthanol. Le milieu réactionnel est agité 20 heures à température ambiante puis l'éthanol est évaporé. La phase aqueuse résiduelle est extraite 2 fois à l'éther, puis acidifiée par 25 cm³ d'acide chlorhydrique à 37 %, saturée de chlorure de sodium, et extraite de nouveau 6 fois à l'éther. Les phases éthérées réunies sont séchées sur sulfate de sodium et concentrées obtenir 41,2 g de produit attendu.

### Stade 5 : 1-[(tert-Butoxycarbonyl)amino]-2,2-diméthylcyclopropanecarboxylate d'éthyle

Sous atmosphère inerte et à température ambiante, une solution de 29,20 g de diphenylphosphorylazide dans 30 cm³ de toluène est additionnée goutte à goutte, à une solution de 19,77 g du composé obtenu au stade 4 précédent, de 15,2 cm³ de triéthylamine, et de 23,6 g de *tert*-butanol dans 150 cm³ de toluène. Le milieu réactionnel est ensuite chauffé 20 heures à 85°C sous agitation. Après refroidissement, le mélange réactionnel est lavé avec 70 cm³ d'une solution aqueuse de carbonate de sodium. La phase aqueuse est extraite 3 fois avec 100 cm³ de toluène. Les phases organiques réunies sont séchées sur sulfate de sodium et concentrées. La chromatographie sur silice (dichlorométhane/tétrahydrofuranne : 98/2) permet d'obtenir 12,75 g de produit attendu.

### Stade 6 : 1-[(tert-Butoxycarbonyl)(méthyl)amino]-2,2-diméthylcyclopropanecarboxylate d'éthyle

16,6 g de *tert*-butylate de potassium sont ajoutés sous agitation, en 1 heure à une solution de 24,1 g de composé obtenu au stade 5 précédent dans 240 cm³ de diméthylformamide. Le milieu réactionnel est agité 30 minutes à température ambiante puis 20,0 g d'iodure de méthyle sont additionnés goutte à goutte. Le mélange est agité à température ambiante pendant 2 heures. Le milieu réactionnel est concentré à sec et le résidu est repris dans 300 cm³ d'éther. La phase organique est lavée avec 40 cm³ d'une solution aqueuse d'hydrogénocarbonate de sodium et avec une solution aqueuse de chlorure de lithium (2 x 20 cm³), puis séchée sur sulfate de sodium et concentrée. La chromatographie sur silice (dichlorométhane), permet d'obtenir 17,6 g de produit attendu.

### Stade 7 : 2,2-Diméthyl-1-(méthylamino)cyclopropanecarboxylate d'éthyle

270 cm³ d'une solution d'acide chlorhydrique 8,7 N dans l'éthanol sont additionnés goutte à goutte à une solution, refroidie à 5°C, de 18,06 g du composé obtenu au stade 6 précédent dans 60 cm³ d'éthanol. Le milieu réactionnel est agité 3 heures à température ambiante, puis concentré à sec. Le résidu est repris dans l'éthanol puis concentré de nouveau à sec, 3 fois de suite. Une solution aqueuse d'hydrogénocarbonate de sodium est ajoutée au chlorhydrate obtenu et la phase aqueuse est extraite 5 fois au dichlorométhane. Les phases organiques réunies sont séchées sur sulfate de sodium puis concentrées. On isole 10,94 g de produit attendu.

### Stade 8 : 1-[Benzoyl(méthyl)amino]-2,2-diméthylcyclopropanecarboxylate d'éthyle

Une solution de 9,88 g de chlorure de benzoyle dans 40 cm³ de tétrahydrofuranne est additionnée goutte à goutte à une solution, refroidie à 5°C, de 10,94 g du composé obtenu au stade 7 précédent et 7,76 g de triéthylamine dans 170 cm³ de tétrahydrofuranne. Le milieu réactionnel est agité 45 minutes à 5°C puis 2 heures à température ambiante. Le précipité blanc formé est filtré et lavé au THF. Le filtrat est concentré à sec. Le résidu obtenu est dilué dans du dichlorométhane et lavé avec une solution aqueuse saturée en carbonate de sodium. Après décantation, la phase aqueuse est extraite 4 fois au dichlorométhane. Les phases organiques jointes sont ensuite séchées sur sulfate de sodium et concentrées à sec. Une chromatographie sur silice (dichlorométhane/tétrahydrofuranne : 95/5), permet d'obtenir 16,07 g de produit attendu.

### Stade 9 : {1-[Benzyl(méthyl)amino]-2,2-diméthylcyclopropyl}méthanol

Une solution de 16,07 g du composé obtenu au stade 8 précédent dans 130 cm³ de tétrahydrofuranne est additionnée goutte à goutte à une suspension de 4,44 g d'hydrure d'aluminium lithium dans 350 cm³ de tétrahydrofuranne. Le mélange est porté à reflux pendant 4 heures. Il est ensuite refroidi à 5°C puis hydrolysé par 4,5 cm³ d'eau distillée, 4,5 cm³ d'hydroxyde de sodium 4N et 15 cm³ d'eau distillée. Après 30 minutes d'agitation à température ambiante, un précipité est filtré et rincé au tétrahydrofuranne. Le filtrat est séché sur sulfate de sodium et concentré à sec. Une chromatographie sur silice (dichlorométhane (100) à dichlorométhane/tétrahydrofuranne : 95/5), permet d'isoler 11,9 g de produit attendu.

### Stade 10 : N-Benzyl-N,2,2-triméthyl-1-[(3-pyridinyloxy)méthyl]cyclopropanamine

1,86 g de *tert*-butylate de potassium sont ajoutés à une solution de 3,2 g du produit obtenu au stade 9 précédent dans 96 cm3 de diméthyle sulfoxyde. Après quelques secondes d'agitation, 6,3 cm³ de 3-fluoropyridine sont ajoutés. Le milieu réactionnel est chauffé 2 minutes au reflux dans un four à micro-ondes multimode de 1000 W. Après refroidissement, le milieu réactionnel est coulé sur 300 cm³ de solution aqueuse saturée en chlorure de sodium, puis extrait 6 fois à l'éther. Les phases organiques réunies sont lavées avec une solution de chlorure de sodium puis séchées sur sulfate de sodium et évaporées. Une chromatographie sur silice (dichlorométhane), du résidu obtenu permet d'obtenir 3,83 g de produit attendu.

### Stade 11 : Dichlorhydrate de (R,S)-N,2,2-triméthyl-1-[(3-pyridinyloxy)méthyl]cyclopropanamine

8,8 cm³ d'acide chlorhydrique 1 N sont ajoutés à une solution de 1,35 g du composé obtenu au stade 10 précédent dans 40 cm³ d'éthanol. Le milieu réactionnel est hydrogéné 5 heures sous pression atmosphérique, à température ambiante, en présence de 0,27 g d'hydroxyde de palladium (20% sur charbon). Le catalyseur est filtré et les solvants évaporés. Le résidu est repris dans 30 cm³ de solution aqueuse saturée en carbonate de sodium, puis extrait à l'éther. Les phases éthérées sont séchées sur sulfate de sodium puis concentrées à sec. 20 cm³ d'une solution d'acide chlorhydrique 4N dans l'éther sont ajoutés au résidu et le chlorhydrate formé est filtré. Après séchage, on obtient 0,79 g du composé attendu.
Spectrométrie de masse (ESI) : m/z = 207,1 Th ([M+H]⁺)
Point de fusion (cap) : 86-88°C

### Stade 12 : Séparation des énantiomères du composé de l'exemple 25

La séparation des énantiomères de 2,80 g du produit racémique obtenu au stade 10 de l'exemple 25 a été réalisé par chromatographie chirale sur colonne Chiracel OJ (éthanol). On isole ainsi 1,43 g du premier énantiomère α et 1,37 g du second énantiomère β.

### énantiomère α :

### (R ou S)-(+)-N-Benzyl-N,2,2-triméthyl-1-[(3-pyridinyloxy)méthyl]cyclopro-panamine ou (R ou S)-(-)-N-Benzyl-N,2,2-triméthyl[(3-pyridinyloxy)-méthyl cyclopropanamine

### énantiomère β :

### (R ou S)-(+)-N-Benzyl-N,2,2-triméthyl-1-[(3-pyridinyloxy)méthyl]cyclopro-panamine ou (R ou S)-(-)-N-Benzyl-N,2,2-triméthyl-1-[(3-pyridinyloxy)-méthyl] cyclopropanamine

rapport énantiomérique (énantiomère α) : 97,2/2,8
rapport énantiomérique (énantiomère β) : 99,1/0,9

### Exemple 26 (énantiomère α) :

### Difumarate de (R ou S)-(+)-N,2,2-triméthyl-1-[(3-pyridinyloxy)-méthyl] cyclopropanamine

La débenzylation de l'énantiomère α obtenus au stade 12 de l'exemple 25 a été réalisée selon la procédure décrite au stade 11 de l'exemple 25. Le fumarate de 1,4 g de l'énantiomère α est obtenu par dissolution de la base dans l'éthanol en présence de 1,5 équivalent d'acide fumarique. La solution est concentrée à sec puis reprise dans l'éther. 0,83 g de fumarate est obtenu par filtration et séchage.
Spectrométrie de masse (ESI) : m/z = 207,1 Th ([M+H]⁺)
Point de fusion (cap) : 124-127°C
Pouvoir rotatoire : [α]_{D}²⁰ : +13,5° (c=0,01249 g/cm³, CH₃OH).

### Exemple 27 (énantiomère β) :

### Difumarate de (R ou S)-(-)-N,2,2-triméthyl-1-[(3-pyridinyloxy)-méthyl] cyclopropanamine

Le composé est obtenu selon le procédé de l'exemple 27 en remplaçant l'énantiomère α par l'énantiomère β.
Spectrométrie de masse (ESI) : m/z = 207,1 Th ([M+H]⁺)
Point de fusion (cap) : 115-118°C
Pouvoir rotatoire : [α]_{D}²⁰: -14,1° (c=0,0099 g/cm³, CH₃OH)

### ETUDES PHARMACOLOGIQUES DES COMPOSES DE L'INVENTION

### Exemple A :

### Déplacement de la fixation de l'[¹²⁵I]-α-bungarotoxine sur les récepteurs nicotiniques de l'organe électrique de torpille

Cette étude, réalisée selon la méthode décrite dans J. Pharmacol. Exp. Ther., 1994, 271 ; 624-631, a pour objectif d'évaluer l'affinité des composés de la présente invention sur les récepteurs nicotiniques de « type musculaire ».

Des membranes (1-5 µg/ml) d'organe électrique de torpille sont incubées (1h, 22°C) en présence d'une gamme de concentration (0,01-10 µM) de chaque composé de l'invention (dilution à partir d'une solution mère à 10 mM dans du DMSO) en présence d'[¹²⁵I]-α-bungarotoxine (A.S. : 7,4 TBq/mmol : 0,2 nM) dans du tampon Krebs (Tris-HCl 50 mM, KCl 5 mM, MgCl₂ 1 mM, CaCl₂ 2 mM, NaCl 100 mM, pH 7.4) avec 0,01 % de BSA, volume final de 500 µl. La liaison non-spécifique est déterminée par l'incubation de membranes en présence d'α-bungarotoxine (1 µM).

Les résultats indiquent que tous les composés de la présente invention ne possèdent aucune affinité significative vis-à-vis des récepteurs nicotiniques de « type musculaire », jusqu'à la concentration de 10 µM (Kᵢ > 10⁻⁵M).

### Exemple B :

### Déplacement de la fixation d'[³H]-épibatidine sur les récepteurs nicotiniques de cellules IMR32

Cette étude, réalisée selon la technique décrite dans Molec. Pharmacol., 1995, 48 ; 280-287, a pour but de déterminer l'affinité des composés de la présente invention sur les récepteurs nicotiniques de « type ganglionnaire » (American Soc. Neuroscience, 2000, 26, 138).

Des membranes (250 µg/ml) de cellules neuroblastome IMR-32 sont incubées (2h, 20°C) en présence d'une gamme de concentration (0,01-10 µM) de chaque composé de l'invention (dilution à partir d'une solution mère à 10 mM dans du DMSO) et d'(±)-['H]-épibatidine (A.S.: 2464 GBq/mmol: 1,5 nM) dans du tampon phosphate (NaH₂PO₄ 20 mM, pH 7,4), volume final de 250 µl. La liaison non-spécifique est déterminée par l'incubation de membranes en présence de 300 µM de (-)nicotine.

Les résultats montrent que tous les composés de la présente invention n'ont aucune affinité significative sur les récepteurs nicotiniques de «type ganglionnaire », jusqu'à la concentration de 10 µM (Kᵢ > 10⁻⁵M).

### Exemple C :

### Déplacement de la fixation d'[³H]-oxotrémorine-M sur les récepteurs muscariniques de cerveau de rat

Cette étude, réalisée selon la méthode décrite dans Naumyn-Schmiederberg's Arch. Pharmacol., 2001, 363, 429-438, a pour objectif de déterminer l'affinité des composés de la présente invention sur les récepteurs muscariniques.

Des membranes (250 µg/ml) de cerveau de rat sont incubées (2h, 20°C) en présence d'une gamme de concentration (0,01-10 µM) de chaque composé de l'invention (dilution à partir d'une solution mère à 10 mM dans du DMSO) et d'[³H]-oxotrémorine-M (A.S. : 3174 GBq/mmol : 2 nM) dans du tampon phosphate (NaH₂PO₄ 20 mM, pH 7,4), volume final de 250 µl. La liaison spécifique est déterminée par l'incubation des membranes en présence d'atropine (1 µM). L'affinité des composés de la présente invention vis-à-vis des récepteurs muscariniques est caractérisée par la détermination du Kᵢ.

Les résultats démontrent que tous les composés de la présente invention, jusqu'à la concentration de 10 µM, sont dépourvus d'affinité vis-à-vis des récepteurs muscariniques (Kᵢ > 10⁻⁵M).

### Exemple D :

### Déplacement de la fixation de l'[¹²⁵I]-α-bungarotoxine sur les récepteurs nicotiniques de « type α7» de cerveau de rat

Cette étude, réalisée selon la méthode décrite dans Molec. Pharmacol., 1986, 30 ; 427-436, a pour objectif de déterminer l'affinité des composés de la présente invention vis-à-vis des récepteurs centraux nicotiniques de type α7.

Des membranes (1000 µg/ml) de cerveau de rat sont incubées (5h, 37°C) en présence d'une gamme de concentration (0,01-10 µM) de chaque composé de la présente invention (dilution à partir d'une solution mère à 10 mM dans du DMSO) et d'[¹²⁵I]-α-bungarotoxine (A.S. : 7,4 TBq/mmol : 1 nM) dans du tampon Krebs (Tris-HCl 50 mM, KC15 mM, MgCl₂ 1 mM, CaCl₂ 2 mM, NaCl 100 mM, pH 7,4) avec 0,05 % de BSA, volume final de 500 µl. La liaison non-spécifique est déterminée par l'incubation de membranes en présence d'α-bungarotoxine (1 µM).

L'affinité des composés de la présente invention vis-à-vis des récepteurs nicotiniques de type α7 est caractérisée par la détermination du Kᵢ.

Les résultats indiquent que tous les composés de la présente invention, jusqu'à la concentration de10 µM, sont dépourvus d'affinité vis-à-vis des récepteurs nicotiniques centraux de type α7 (Kᵢ > 10⁻⁵M).

### Exemple E :

### Déplacement de la fixation de [³H]-cytisine sur les récepteurs nicotiniques de « type α4β2 » de cerveau de rat

Cette étude, réalisée selon la technique décrite dans Molec. Pharmacol., 1990, 39 ; 9-12, a pour objectif de déterminer l'affinité des composés de la présente invention vis-à-vis des récepteurs nicotiniques centraux de type α4β2.

Des membranes (250 µg/ml) de cerveau de rat sont incubées (2h, 20°C) en présence d'une gamme de concentration (0,01-10 µM) de chaque composé de la présente invention (dilution à partir d'une solution mère à 10 mM dans du DMSO) et de [³H]-cytisine (A.S. : 1184 GBq/mmol : 2 nM) dans du tampon phosphate (NaH₂PO₄ 20 mM, pH 7,4), volume final de 250µl. La liaison non-spécifique est déterminée par l'incubation de membranes en présence de 10 µM de (-)nicotine. L'affinité des composés de la présente invention vis-à-vis des récepteurs nicotiniques centraux de type α4β2 est caractérisée par la détermination du Kᵢ.

Les résultats obtenus montrent que les composés de la présente invention présentent une forte affinité vis-à-vis des récepteurs nicotiniques centraux de type α4[32. Ainsi, l'exemple 2 présente un Kᵢ de 6.9 x 10⁻⁸M.

Ces résultats, ainsi que ceux obtenus dans les exemples A à D indiquent que les composés de la présente invention sont de puissants ligands nicotiniques centraux spécifiques des récepteurs de type α4β2.

### Exemple F :

### Mesure in vivo de la libération d'acétylcholine par microdialyse intra-corticale chez le rat Wistar vigile

L'administration systémique de nicotine et d'agonistes nicotiniques induit une augmentation *in vivo* d'acétylcholine dans diverses régions cérébrales (Neurochem. Res., 1996, 21, 1181-1186 ; Eur. J. Pharmacol., 1998, 351, 181-188 ; Br. J. Pharmacol., 1999, 127, 1486-1494). Une sonde de microdialyse est implantée au niveau du cortex préfrontal médian de rats mâles Wistar. Six ou sept jours après l'implantation des sondes, celles-ci sont perfusées par du Ringer (NaCl 147 mM, KCl 2.7 mM, CaCl₂ 1.2 mM, MgCl₂ 1 mM, néostigmine 20 nM) à un débit de 1 µl/min chez l'animal libre de se mouvoir. Après 2 heures de stabulation, le produit étudié est administré par voie intrapéritonéale. Un groupe d'animaux témoins reçoit le solvant du produit. Puis, les dialysats (30 µl) sont collectés toutes les 30 minutes pendant 4h afin de mesurer les concentrations extra-synaptiques corticales d'acétylcholine par HPLC en détection ampérométrique. Les résultats sont exprimés en pg d'acétylcholine/dialysat et les comparaisons inter-groupes sont effectuées par une analyse de variance à 2 facteurs (traitement x temps) avec mesures répétées sur le temps.

Les résultats obtenus montrent que les composés de la présente invention augmentent la libération corticale *in vivo* d'acétylcholine de manière dose-dépendante pour des doses allant de 1 à 10 mg/kg IP et indiquent le caractère agoniste α4β2 des composés de la présente invention. Ainsi, l'exemple 2, une heure après administration à la dose de 3 mg/km IP, induit une augmentation de +86 % de la libération d'acétylcholine dans le cortex préfrontal chez le rat Wistar vigile.

### Exemple G :

### Torsions abdominales induites à la phényl-p-benzoquinone (PBQ) chez la souris NMRI

L'administration intrapéritonéale d'une solution alcoolique de PBQ provoque des crampes abdominales chez la Souris (Proc. Soc. Exp. Biol., 1957, 95, 729-731). Ces crampes sont caractérisées par des contractions répétées de la musculature abdominale, accompagnées d'une extension des membres postérieurs. La plupart des analgésiques antagonisent ces crampes abdominales (Brit. J . Pharmacol. Chem., 1968, 32, 295-310). A t=0 min., les animaux sont pesés et le produit étudié est administré par voie IP. Un groupe d'animaux témoins reçoit le solvant du produit. A t=30 min., une solution alcoolique de PBQ (0.2 %) est administrée par voie IP sous un volume de 0,25 ml/souris. Immédiatement après l'administration de la PBQ, les animaux sont placés dans des cylindres en plexiglass (L = 19,5 cm ; D.I. = 5 cm). De t=35 min. à t=45 min., la réaction des animaux est observée et l'expérimentateur note le nombre total de crampes abdominales par animal. Les résultats sont exprimés par le pourcentage d'inhibition du nombre de crampes abdominales mesuré chez les animaux témoins à la dose active du composé étudié.

Les résultats obtenus montrent une inhibition de l'ordre de 70 %, pour des doses actives de 10 mg/kg IP. Ceci montre que les composés de l'invention sont pourvus de propriétés antalgiques. Ainsi, l'exemple 15, administré à la dose de 10 mg/kg IP, diminue de -69 % le nombre de crampes abdominales provoquées par l'administration de PBQ chez la souris.

### Exemple H :

### Reconnaissance sociale chez le Rat Wistar

Initialement décrit en 1982 (J. Comp. Physiol., 1982, 96, 1000-1006), le test de la reconnaissance sociale a été ensuite proposé par différents auteurs (Psychopharmacology, 1987, 91, 363-368 ; Psychophamacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester par voie intrapéritonéale et, 2 heures plus tard, est remis en présence (5 minutes) du jeune rat. Le comportement de reconnaissance sociale est alors à nouveau observé et la durée en est mesurée. Le critère de jugement est la différence (T2-T1), exprimée en secondes, des temps de « reconnaissance » des 2 rencontres.

Les résultats obtenus montrent une différence (T2-T1) comprise entre -22 et -38 s pour des doses allant de 1 à 10 mg/kg IP. Ceci montre que les composés de l'invention augmentent la mémorisation de façon très importante, et à faible dose. Ainsi, l'exemple 2, à la dose de 10 mg/kg IP, induit une différence (T2-T1) de 38 s.

### Exemple I :

### Compositions pharmaceutiques pour 1000 comprimés dosés à 10 mg

| | |
|---|---|
| Composé de l'exemple 2 | 10 g |
| Hydroxypropylméthylcellulose | 10 g |
| Amidon de blé | 15 g |
| Lactose | 90 g |
| Stéarate de magnésium | 2 g |

## Revendications

1. Composés de formule (I) : 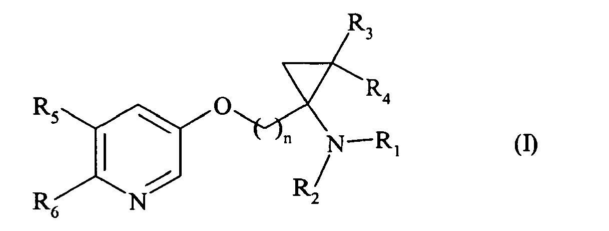 dans laquelle :
n représente un nombre entier compris entre 1 et 6 inclus,
R₁ et R₂, identiques ou différents, indépendamment l'un de l'autre, représentent un atome d'hydrogène, ou un groupement alkyle (C₁-C₆) linéaire ou ramifié ou arylalkyle (C₁-C₆) linéaire ou ramifié,
R₃ et R₄, identiques ou différents, indépendamment l'un de l'autre, représentent un atome d'hydrogène, ou un groupement alkyle (C₁-C₆) linéaire ou ramifié, étant entendu qu'au moins un des deux groupements R₃ ou R₄ représente un groupement alkyle (C₁-C₆) linéaire ou ramifié,
R₅ et R₆, identiques ou différentes, indépendamment l'un de l'autre, représentent un atome d'hydrogène, ou un groupement alkyle (C₁-C₆) linéaire ou ramifié, halogène, hydroxy, alkoxy (C₁-C₆) linéaire ou ramifié, cyano, nitro, acyle (C₂-C₆) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, ou amino éventuellement substitué par un ou deux groupements alkyle (C₁-C₆) linéaire ou ramifié,
par groupement aryle, on comprend un groupement phényle, biphényle, naphtyle, dihydronaphtyle, tétrahydronaphtyle, indanyle et indényle, chacun de ces groupements étant éventuellement substitués par un ou plusieurs groupements, identiques ou différents, choisis parmi halogène, alkyle (C₁-C₆) linéaire ou ramifié, hydroxy, cyano, nitro, alkoxy (C₁-C₆) linéaire ou ramifié, acyle (C₂-C₇) linéaire ou ramifié, alkoxycarbonyle (C₁-C₆) linéaire ou ramifié, trihalogénoalkyle (C₁-C₆) linéaire ou ramifié, trihalogénoalkoxy (C₁-C₆) linéaire ou ramifié, et amino éventuellement substitué par un ou deux groupements alkyle (C₁-C₆) linéaire ou ramifié, leurs énantiomères, diastéréoisomères ainsi que leurs sels d'addition à un acide ou à une base pharmaceutiquement acceptable.

2. Composés de formule (I) selon la revendication 1, **caractérisés en ce que** n est un entier prenant la valeur 1, leurs énantiomères, diastéréoisomères ainsi que leurs sels d'addition à un acide ou à une base pharmaceutiquement acceptable.

3. Composés de formule (I) selon la revendication 1, **caractérisés en ce que** R₁ et R₂, identiques ou différents, représentent chacun un atome d'hydrogène ou un groupement alkyle (C₁-C₆) linéaire ou ramifié, leurs énantiomères, diastéréoisomères ainsi que leurs sels d'addition à un acide ou à une base pharmaceutiquement acceptable.

4. Composés de formule (I) selon la revendication 1, **caractérisés en ce que** R₃ et R₄, identiques ou différents, représentent chacun un atome d'hydrogène ou un groupement méthyl, leurs énantiomères, diastéréoisomères ainsi que leurs sels d'addition à un acide ou à une base pharmaceutiquement acceptable.

5. Composés de formule (I) selon la revendication 1, **caractérisés en ce que** R₅ et R₆, identiques ou différents, représentent chacun un atome d'hydrogène ou un atome d'halogène, leurs énantiomères, diastéréoisomères ainsi que leurs sels d'addition à un acide ou à une base pharmaceutiquement acceptable.

6. Composés de formule (I) selon la revendication qui sont le :
(1*S*,2*S*), (1*R*,2*R*)-*N*,2-diméthyl-1-[(3-pyridinyloxy)méthyl]cyclopropanamine
(1*S*,2*S*) ou (1*R*,2*R*)-(-)-*N*,2-diméthyl-1-[(3-pyridinyloxy)méthyl]cyclopropanamine
(1*S*,2*S*) ou (1*R*,2*R*)-(+)-*N*,2-diméthyl-1-[(3-pyridinyloxy)méthyl]cyclopropanamine
(1*S*,2*S*), (1*R*,2*R*)-2-Méthyl-1-[(3-pyridinyloxy)méthyl]cyclopropanamine
(1*S*,2*S*) ou (1*R*,2*R*)-(-)-2-méthyl-1-[(3-pyridinyloxy)méthyl]cyclopropanamine
(1*S*,2*S*) ou (1*R*,2*R*)-(+)-2-méthyl-1-[(3-pyridinyloxy)méthyl]cyclopropanamine
(1*S*,2*S*), (1*R*,2*R*)-*N*,*N*,2-Triméthyl-1-[(3-pyridinyloxy)méthyl]cyclopropanamine
(1*S*,2*S*) ou (1*R*,2*R*)-(+)-*N*,*N*,2-triméthyl-1-[(3-pyridinyloxy)méthyl]-cyclopropanamine
(1*S*,2*S*) ou (1*R*,2*R*)-(-)-*N*,*N*,2-triméthyl-1-[(3-pyridinyloxy)méthyl]-cyclopropanamine
(1*S*,2*S*), (1*R*,2*R*)-1-{[(6-Chloro-3-pyridinyl)oxy]méthyl}-*N*,2-diméthylcyclopropanamine
(1*S*,2*S*) ou (1*R*,2*R*)-(-)-1-{[(6-chloro-3-pyridinyl)-oxy]méthyl}-*N*,2-diméthylcyclopro-panamine
(1*S*,2*S*) ou (1*R*,2*R*)-(+)-1-{[(6-chloro-3-pyridinyl)-oxy]méthyl}-*N*,2-diméthylcyclopro-panamine
(1*S*,2*S*), (1*R*,2*R*)-1-{[(6-Chloro-3-pyridinyl)oxy]méthyl}-2-méthylcyclopropanamine
(1*S*,2*S*) ou (1*R*,2*R*)-(-)-1-{[(6-chloro-3-pyridinyl)-oxy]méthyl}-2-méthylcyclopropanamine
(1*S*,2*S*) ou (1*R*,2*R*)-(+)-1-{[(6-chloro-3-pyridinyl)-oxy]méthyl}-2-méthylcyclopro-panamine
(1*S*,2*S*), (1*R*,2*R*)-1-{[(6-Chloro-3-pyridinyl)oxy]méthyl}-*N*,*N*,2-triméthylcyclopro-panamine
(1*S*,2*S*) ou (1*R*,2*R*)-(+)-1-{[(6-chloro-3-pyridinyl)-oxy]méthyl}-*N*,*N,*2-triméthylcyclopro-panamine
(1*S*,2*S*) ou (1*R*,2*R*)-(-)-1-{[(6-chloro-3-pyridinyl)-oxy]méthyl}-*N*,*N*,2-triméthylcyclopro-panamine
(1*S*,2*R*), (1*R*,2*S*)-*N*,2-Diméthyl-1-[(3-pyridinyloxy)méthyl]-cyclopropanamine
(1*S*,2*R*) ou (1*R*,2*S*)-(+)-*N*,2-diméthyl-1-[(3-pyridinyloxy)méthyl]cyclopropanamine
(1*S*,2*R*) ou (1*R*,2*S*)-(-)-*N*,2-diméthyl-1-[(3-pyridinyloxy)méthyl]cyclopropanamine
(1*S*,2*R*), (1*R*,2*S*)-1-{[(5-bromo-3-pyridinyl)oxy]méthyl}-*N*,2-diméthylcyclopropanamine
(1*S*,2*R*), (1*R*,2*S*)-2-méthyl-1-[(3-pyridinyloxy)méthyl]cyclopropanamine
(1*S*,2*R*), (1*R*,2*S*)-*N*,*N*,2-triméthyl-1-[(3-pyridinyloxy)méthyl]cyclopropanamine
(*R*,*S*)-*N*,2,2-triméthyl-1-[(3-pyridinyloxy)méthyl]cyclopropanamine
(R ou *S*)-(+)-*N*,2,2-triméthy)-1-[(3-pyridinyloxy)-méthyl]cyclopropanamine
(R ou *S*)-(-)-*N*,2,2-triméthyl-1-[(3-pyridinyloxy)-méthyl]cyclopropanamine,
leurs énantiomères, diastéréoisomères ainsi que leurs sels d'addition à un acide ou à une base pharmaceutiquement acceptable.

7. Procédé de préparation des composés de formule (I) selon la revendication 1, **caractérisé en ce qu'**on utilise comme produit de départ un composé de formule (II) : 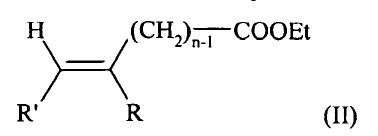 dans lequel R représente un groupement choisi parmi éthoxycarbonyle, cyano et isocyano, R' représente un groupement choisi parmi méthyle et 2-bromopropyle, composé de formule (II), qui sont mis à réagir avec de l'iodure de triméthylsulfoxonium en présence d'hydrure de sodium dans du diméthyle sulfoxyde, pour conduire aux composés de formule (III) : 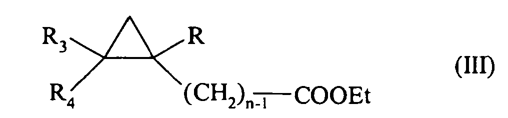 dans laquelle R est tel que défini précédemment et R₃, R₄ et n sont tels que définis dans la formule (I),
composés de formule (III), qui sont :
- soit mis en présence d'hydroxyde de sodium dans l'éthanol lorsque R représente un groupement éthoxycarbonyle, pour conduire aux composés de formule (IV) : 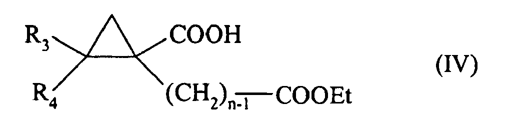
dans laquelle R₃, R₄ et n sont tels que définis précédemment,
composés de formule (IV), qui sont mis à réagir avec du diphénylphosphorylazide et du tert-butanol dans le toluène, en présence de triéthylamine, pour conduire aux composés de formule (V) : 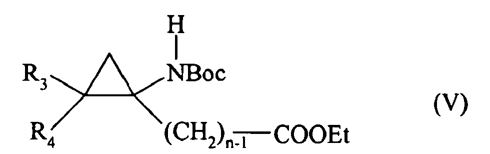 dans laquelle R₃, R₄ et n sont tels que définis précédemment et Boc représente le groupement tert-butoxycarbonyl, composés de formule (V) qui est mis à réagir avec un composé de formule (VI) :
R'₁-L₁ (VI)
dans laquelle R'₁ représente un groupement choisi parmi alkyle (C₁-C₆) linéaire ou ramifié et arylalkyle (C₁-C₆) linéaire ou ramifié, et L₁ représente un groupement partant usuel de la chimie organique, en présence de tert-butylate de potassium dans du diméthylformamide, pour conduire aux composés de formule (VII) : 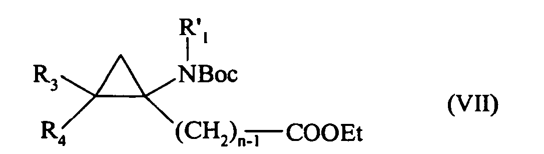 dans laquelle R₃, R₄, R'₁, Boc et n sont tels que définis précédemment,
l'ensemble des composés de formules (V) et (VII) formant les composés de formule (VIII) : 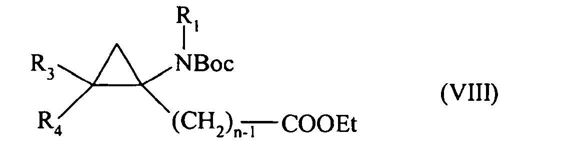 dans laquelle R₃, R₄, Boc et n sont tels que définis précédemment et R₁ est tel que défini dans la formule (I),
composés de formule (VIII), qui sont :
* soit mis en présence d'un agent réducteur dans un solvant anhydre, pour conduire aux composés de formule (IX) : 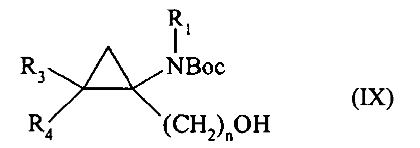
dans laquelle R₃, R₄, Boc, n et R₁ sont tels que définis précédemment,
composés de formule (IX) qui sont soit mis à réagir avec du triphénylphosphine et du tétrabromométhane dans l'éther, pour conduire aux composés de formule (X) : 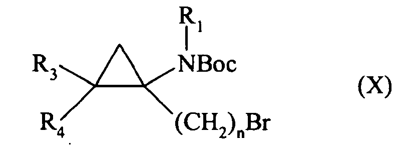 dans laquelle R₃, R₄, Boc, n et R₁ sont tels que définis précédemment,
composés de formule (X) qui sont mis à réagir avec un composé de formule (XI) : 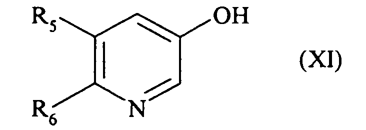 dans lequel R₅ et R₆ sont tels que définis dans la formule (I), en présence de carbonate de césium dans la butanone, pour conduire aux composés de formule (XII) : 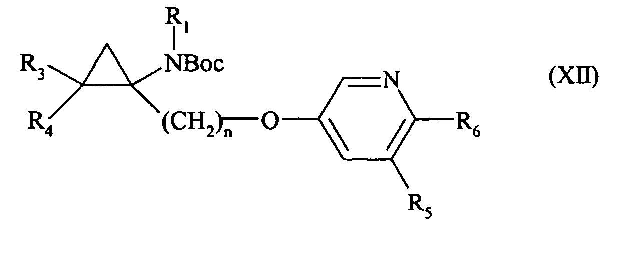 dans laquelle R₃, R₄, R₅, R₆, R₁, n et Boc sont tels que définis précédemment,
composés de formule (XII) qui est mis en présence d'acide chlorhydrique dans le dioxanne pour conduire aux composés de formule (I/a), cas particulier des composés de formule (I) : 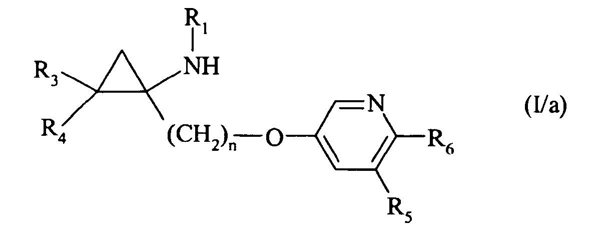 dans laquelle R₁, R₃, R₄, R₅, R₆ et n sont tels que définis précédemment,
composés de formule (I/a) qui sont :
- soit traités par de l'acide formique et une solution aqueuse de formaldéhyde, pour conduire aux composés de formule (I/b), cas particulier des composés de formule (I) : 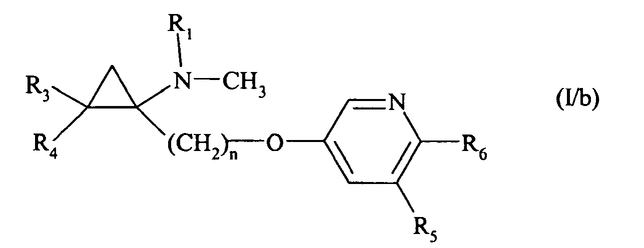
dans laquelle R₁, R₃, R₄, R₅, R₆ et n sont tels que définis précédemment,
- soit mis à réagir avec un composé de formule (XIII) :
R'₂ -L₁ (XIII)
dans laquelle L₁ est tel que défini précédemment et R'₂ représente un groupement choisi parmi alkyle (C₁-C₆) linéaire ou ramifié et arylalkyle (C₁-C₆) linéaire ou ramifié, dans les mêmes conditions que les composés de formule (V), pour conduire aux composés de formule (I/c), cas particulier des composés de formule (I) : 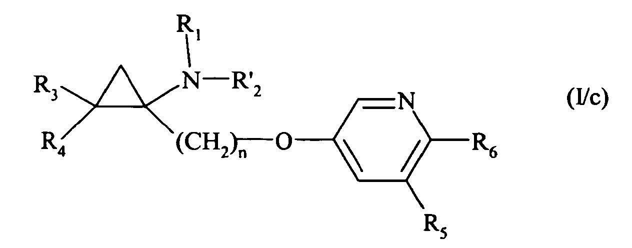
dans laquelle R₁, R₃, R₄, R₅, R₆, n et R'₂ sont tels que définis précédemment,
ou composés de formule (VIII) qui sont :
* soit traités par de l'acide formique et une solution aqueuse de formaldéhyde pour conduire aux composés de formule (XIV) : 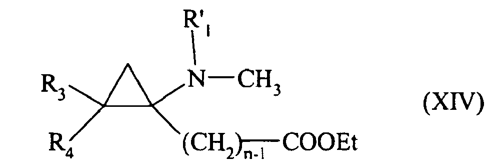
dans laquelle R'₁, R₃, R₄ et n sont tels que définis précédemment,
* soit successivement :
- traités par de l'acide chlorhydrique dans du dioxanne
- puis par un composé de formule (XIII) tel que définis précédemment, dans les mêmes conditions que les composés de formule (V) pour conduire aux composés de formule (XV) : 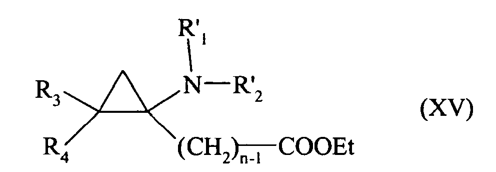
dans laquelle R'₁, R'₂, R₃, R₄ et n sont tels que définis précédemment,
composés de formule (XIV) et (XV) qui sont mis en présence d'un agent réducteur dans un solvant anhydre pour conduire aux composés de formule (XVI) : 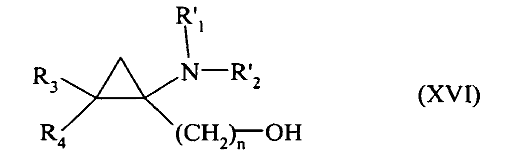 dans laquelle R'₁, R'₂, R₃, R₄ et n sont tels que définis précédemment,
composés de formule (XVI) qui sont mis à réagir avec un composé de formule (XVII) : 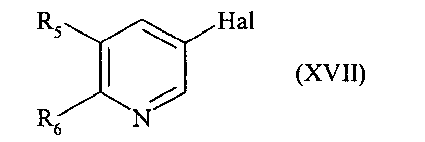 dans lequel R₅ et R₆ sont tels que définis précédemment, et Hal représente un atome de brome ou de fluor, en présence de tert-butylate de potassium dans du DMSO, pour conduire aux composés de formule (I/d), cas particulier des composés de formule (I) : 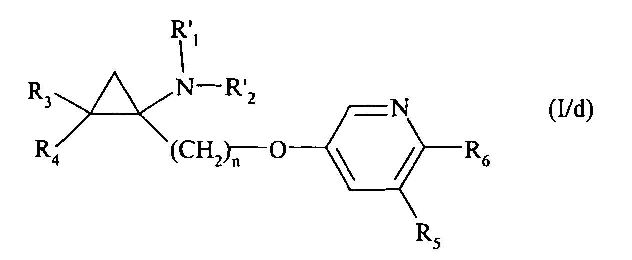 dans laquelle R'₁, R'₂, R₃, R₄, R₅, R₆ et n sont tels que définis précédemment,
composés de formule (I/d) qui est mis à réagir avec de l'acide chlorhydrique en présence d'hydroxyde de palladium dans de l'éthanol lorsque R'₁ représente un groupement benzyle, pour conduire aux composés de formule (I/e), cas particulier des composés de formule (I) : 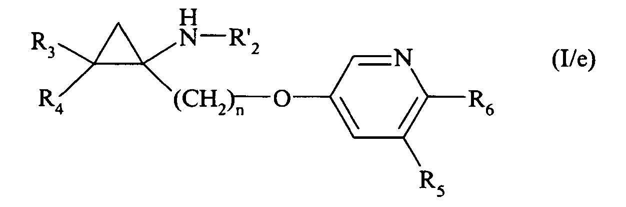 dans laquelle R'₂, R₃, R₄, R₅, R₆ et n sont tels que définis précédemment,
ou composés de formule (VIII) qui sont :
* soit successivement :
- traités par de l'acide chlorhydrique dans l'éthanol
- puis une solution aqueuse d'hydrogénocarbonate de sodium pour conduire aux composés de formule (XVIII) : 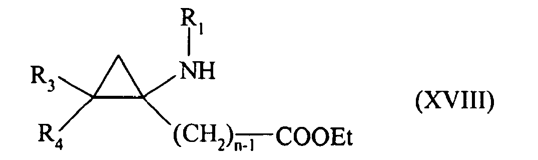
dans laquelle R₁, R₃, R₄ et n sont tels que définis précédemment,
composés de formule (XVIII) qui sont mis à réagir avec du chlorure de benzoyle en présence de triéthylamine dans du tétrahydrofuranne, pour conduire aux composés de formule (XIX) : 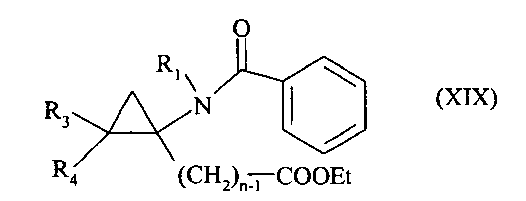 dans laquelle R₁, R₃, R₄ et n sont tels que définis précédemment,
composés de formule (XIX) qui est mis en présence d'un agent réducteur dans un solvant anhydre pour conduire aux composés de formule (XX) : 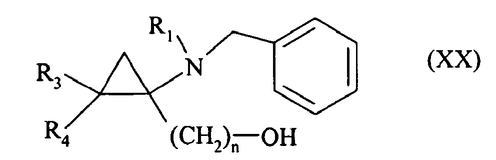 dans laquelle R₁, R₃, R₄ et n sont tels que définis précédemment,
composés de formule (XX) qui est soumis aux mêmes conditions de réaction que les composés de formules (XVI), pour conduire aux composés de formule (I/f), cas particulier des composés de formule (I) : 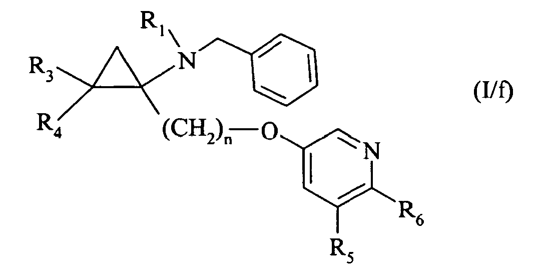 dans laquelle R₁, R₃, R₄, R₅, R₆ et n sont tels que définis précédemment,
composés de formule (I/f) qui sont soumis aux mêmes conditions de réaction que les composés de formule (I/d), pour conduire aux composés de formule (I/a) tel que défini précédemment, cas particulier des composés de formule (I),
ou composés de formule (IX) qui sont :
- soit mis à réagir avec de l'acide chlorhydrique dans du dioxanne, lorsque R₁ représente l'atome d'hydrogène, pour conduire aux composés de formule (XXI) : 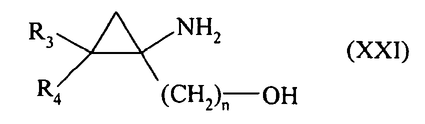
dans laquelle R₃, R₄ et n sont tels que définis précédemment,
composés de formule (XXI) qui sont soumis aux mêmes conditions de réaction que les composés de formule (XVI), pour conduire aux composés de formule (I/g), cas particulier des composés de formule (I), composés de formule (I/g) de stéréochimie cis de (CH₂)ₙ par rapport à l'un des substituants R₃ ou R₄ qui représente un alkyle, l'autre substituant R₃ ou R₄ représente un atome d'hydrogène : 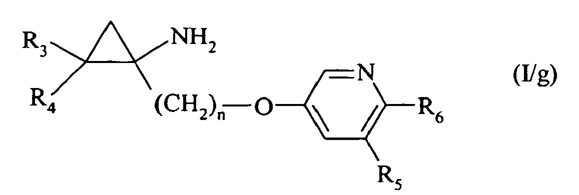 dans laquelle R₃, R₄, R₅, R₆ et n sont tels que définis précédemment,
ou composés de formule (III) qui sont :
- soit mis à réagir avec de l'hydrure d'aluminium lithium dans de l'éther, lorsque R représente un groupement isocyano, pour conduire aux composés de formule (XXII) : 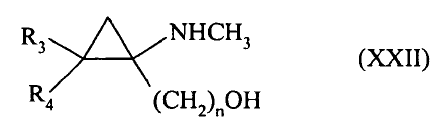
dans laquelle R₃, R₄ et n sont tels que définis précédemment,
composés de formule (XXII) qui sont mis à réagir avec un composé de formule (XVII) tel que défini précédemment, dans les mêmes conditions que les composés de formule (XVI) pour conduire aux composés de formule (I/h), cas particulier des composés de formule (I) : 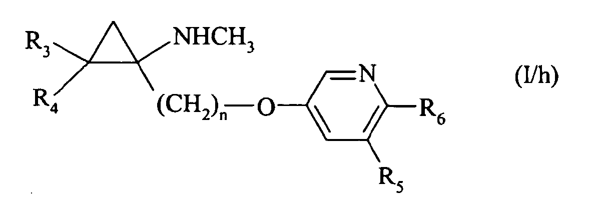 dans laquelle R₃, R₄, R₅, R₆ et n sont tels que définis précédemment,
- soit mis à réagir avec du borohydrure de sodium dans du tétrahydrofuranne, lorsque R représente un groupement cyano, pour conduire aux composés de formule (XXIII) : 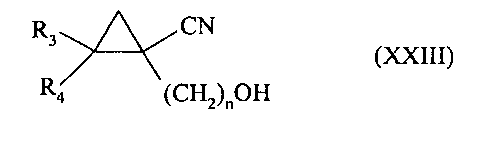
dans laquelle R₃, R₄ et n sont tels que définis précédemment,
composés de formule (XXIII) qui sont mis aux mêmes conditions de réaction que les composés de formule (XVI) pour conduire aux composés de formule (XXIV) : 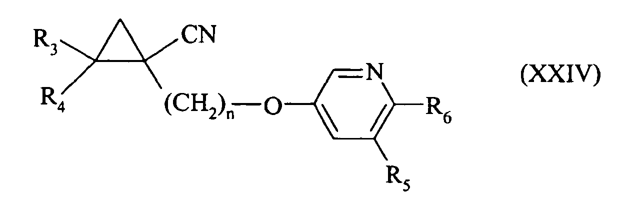 dans laquelle R₃, R₄, R₅, R₆ et n sont tels que définis précédemment,
composés de formule (XXIV) qui sont mis en présence d'une solution aqueuse d'eau oxygénée et d'hydroxyde de lithium dans l'éthanol pour conduire aux composés de formule (XXV) : 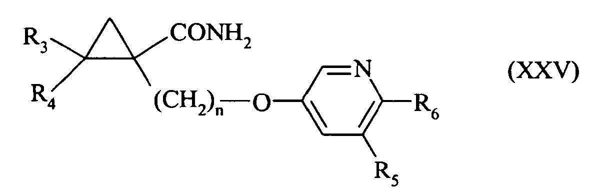 dans laquelle R₃, R₄, R₅, R₆ et n sont tels que définis précédemment,
composés de formule (XXV) qui sont mis à réagir avec du brome et de l'hydroxyde de potassium dans de l'eau, pour conduire aux composés de formule (I/i), cas particulier des composés de formule (I), composés de formule (I/i) de stéréochimie cis de NH₂ par rapport à l'un des substituants R₃ ou R₄ qui représente un alkyle, l'autre substituant R₃ ou R₄ représente un atome d'hydrogène : 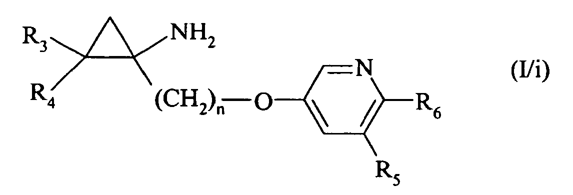 dans laquelle R₃, R₄, R₅, R₆ et n sont tels que définis précédemment,
composés de formule (I/i) qui sont soumis aux mêmes conditions de réaction que les composés de formule (I/a) pour conduire aux composés de formule (I/j), cas particulier des composés de formule (I) : 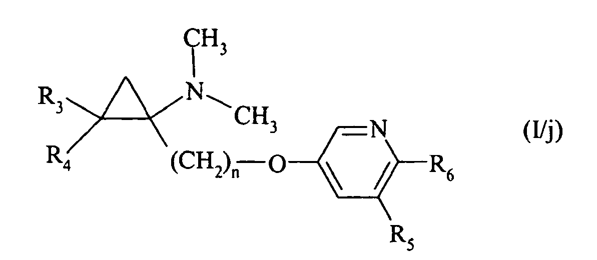 dans laquelle R₃, R₄, R₅, R₆ et n sont tels que définis précédemment,
l'ensemble des composés de formule (I/a) à (I/j) formant l'ensemble des composés de l'invention qui sont purifiés, le cas échéant, selon des techniques classiques de purification, qui peuvent être séparés en leurs différents isomères, selon une technique classique de séparation et qui sont transformés, le cas échéant, en leurs sels d'addition à un acide ou à une base pharmaceutiquement acceptable.

8. Compositions pharmaceutiques contenant comme principe actif au moins un composé selon l'une quelconque des revendications 1 à 6, seul ou en combinaison avec un ou plusieurs excipients ou véhicules inertes, non toxiques, pharmaceutiquement acceptables.

9. Compositions pharmaceutiques selon la revendication 8 contenant au moins un principe actif selon l'une quelconque des revendications 1 à 6 utiles dans le traitement des déficits de mémoire associés au vieillissement cérébral et aux maladies neurodégénératives, ainsi que pour le traitement des troubles de l'humeur, du syndrome de Tourette, du syndrome d'hyperactivité avec déficits attentionnels, du sevrage tabagique et de la douleur.

10. Compositions pharmaceutiques selon la revendication 8 contenant au moins un principe actif selon l'une quelconque des revendications 1 à 6, utiles dans le traitement des déficits de mémoire associés à la maladie d'Alzheimer, la maladie de Parkinson, la maladie de Pick, la maladie de Korsakoff ou les démences frontales et sous-corticales.

## Claims

1. Compounds of formula (I) : 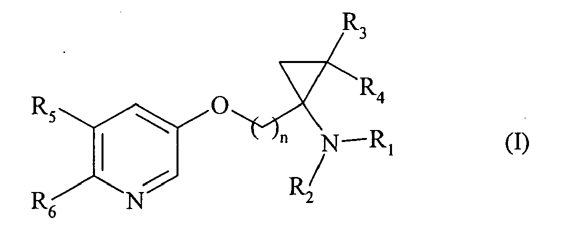 wherein :
n represents an integer of from 1 to 6 inclusive,
R₁ and R₂, which may be identical or different, each independently of the other represent a hydrogen atom, a linear or branched (C₁-C₆)alkyl group or an aryl-(C₁-C₆)alkyl group in which the alkyl moiety may be linear or branched,
R₃ and R₄, which may be identical or different, each independently of the other represent a hydrogen atom or a linear or branched (C₁-C₆)alkyl group, wherein at least one of the two groups R₃ or R₄ represents a linear or branched (C₁-C₆)alkyl group,
R₅ and R₆, which may be identical or different, each independently of the other represent a hydrogen atom or a linear or branched (C₁-C₆)alkyl, halogen, hydroxy, linear or branched (C₁-C₆)alkoxy, cyano, nitro, linear or branched (C₂-C₆)acyl, linear or branched (C₁-C₆)alkoxycarbonyl, linear or branched (C₁-C₆)trihaloalkyl or linear or branched (C₁-C₆)trihaloalkoxy group or an amino group optionally substituted by one or two linear or branched (C₁-C₆)alkyl groups,
there being understood by aryl group a phenyl, biphenyl, naphthyl, dihydronaphthyl, tetrahydronaphthyl, indanyl or indenyl group, each of those groups being optionally substituted by one or more identical or different groups selected from halogen atoms, linear or branched (C₁-C₆)alkyl, hydroxy, cyano, nitro, linear or branched (C₁-C₆)alkoxy, linear or branched (C₂-C₇)acyl, linear or branched (C₁-C₆)alkoxycarbonyl, linear or branched (C₁-C₆)trihaloalkyl and linear or branched (C₁-C₆)trihaloalkoxy groups and amino groups optionally substituted by one or two linear or branched (C₁-C₆)alkyl groups, their enantiomers, diastereoisomers, and also addition salts thereof with a pharmaceutically acceptable acid or base.

2. Compounds of formula (I) according to claim 1, **characterised in that** n is an integer having the value 1, their enantiomers, diastereoisomers, and also addition salts thereof with a pharmaceutically acceptable acid or base.

3. Compounds of formula (I) according to claim 1, **characterised in that** R₁ and R₂, which may be identical or different, each represent a hydrogen atom or a linear or branched (C₁-C₆)alkyl group, their enantiomers, diastereoisomers, and also addition salts thereof with a pharmaceutically acceptable acid or base.

4. Compounds of formula (I) according to claim 1, **characterised in that** R₃ and R₄, which may be identical or different, each represent a hydrogen atom or methyl group, their enantiomers, diastereoisomers, and also addition salts thereof with a pharmaceutically acceptable acid or base.

5. Compounds of formula (I) according to claim 1, **characterised in that** R₅ and R₆, which may be identical or different, each represent a hydrogen atom or a halogen atom, their enantiomers, diastereoisomers, and also addition salts thereof with a pharmaceutically acceptable acid or base.

6. Compounds of formula (I) according to claim which are:
(1*S*,2*S)*, *(1R*,*2R*)-*N*,2-dimethyl-1-[(3-pyridinyloxy)methyl]cyclopropanamine
(1*S*,2*S)*- or *(1R*,2*R*)-(-)-*N*,2-dimethyl-1-[(3-pyridinyloxy)methyl]cyclopropanamine
(1*S*,2*S)*- or *(1R*,2*R*)-(+)-*N*,2-dimethyl-1-[(3-pyridinyloxy)methyl]cyclopropanamine
(1*S*,2*S)*, *(1R*,2*R*)-2-methyl-1-[(3-pyridinyloxy)methyl]cyclopropanamine
(1*S*,2*S)*- or *(1R*,2*R*)-(-)-2-methyl-1-[(3-pyridinyloxy)methyl]cyclopropanamine
(1*S*,2*S)*- or *(1R*,2*R*)-(+)-2-methyl-1-[(3-pyridinyloxy)methyl]cyclopropanamine
(1*S*,2*S), (1R*,2*R*)-*N*,*N*,2-trimethyl-1-[(3-pyridinyloxy)methyl]cyclopropanamine
(1*S*,2*S)*- or *(1R*,2*R*)-(+)-*N*,*N*,2-trimethyl-1-[(3-pyridinyloxy)methyl]cyclopropanamine
(1*S*,2*S*)- or (1*R*,2*R*)-(-)-*N*,*N*,2-trimethyl-1-[(3-pyridinyloxy)methyl]cyclopropanamine
(1*S*,2*S), (1R*,2*R*)-1-{[(6-chloro-3-pyridinyl)oxy]methyl}-*N*,2-dimethylcyclopropanamine
(1*S*,2*S)*- or *(1R*,2*R*)-(-)-1-{[(6-chloro-3-pyridinyl)oxy]methyl}-*N*,2-dimethylcyclo-propanamine
(1*S*,2*S)*- or *(1R*,2*R*)-(+)-1-{[(6-chloro-3-pyridinyl)oxy]methyl}-*N*,2-dimethylcyclo-propanamine
(1*S*,2*S), (1R*,2*R*)-1-{[(6-chloro-3-pyridinyl)oxy]methyl}-2-methylcyclopropanamine
(1*S*,2*S)-* or *(1R*,2*R*)-(-)-1-{[(6-chloro-3-pyridinyl)oxy]methyl}-2-methylcyclopropanamine
(1*S*,2*S)-* or *(1R*,2*R*)-(+)-1-{[(6-chloro-3-pyridinyl)oxy]methyl}-2-methylcyclopropanamine
(1*S*,2*S), (1R*,2*R*)-1-{[(6-chloro-3-pyridinyl)oxy]methyl}-*N*,*N*,2-trimethylcyclopropanamine
(1*S*,2*S)*- or *(1R*,2*R*)-(+)-1-{[(6-chloro-3-pyridinyl)oxy]methyl}-*N*,*N*,2-trimethylcyclo-propanamine
(1*S*,2*S)*- or *(1R*,2*R*)-(-)-1-{[(6-chloro-3-pyridinyl)oxy]methyl}-*N*,*N*,2-trimethylcyclo-propanamine
(1*S*,2*R), (1R*,2*S*)-*N*,2-dimethyl-1-[(3-pyridinyloxy)methyl]cyclopropanamine
(1*S*,2*R*)- or (1*R*,2*S*)-(+)-*N*,2-dimethyl-1-[(3-pyridinyloxy)methyl]cyclopropanamine
(1*S*,2*R*)- or (1*R*,2*S*)-(-)-*N*,2-dimethyl-1-[(3-pyridinyloxy)methyl]cyclopropanamine
(1*S*,2*R)*, *(1R*,2*S*)-1-{[(5-bromo-3-pyridinyl)oxy]methyl}-*N*,2-dimethylcyclopropanamine
(1*S*,2*R)*, *(1R*,2*S*)-2-methyl-1-[(3-pyridinyloxy)methyl]cyclopropanamine
(1*S*,2*R)*, *(1R*,2*S*)-*N*,*N*,2-trimethyl-1-[(3-pyridinyloxy)methyl]cyclopropanamine
(*R*,*S*)-*N*,2,2-trimethyl-1-[(3-pyridinyloxy)methyl]cyclopropanamine
(R or *S*)-(+)-*N*,2,2-trimethyl-1-[(3-pyridinyloxy)methyl]cyclopropanamine
(R or *S*)-(-)-*N*,2,2-trimethyl-1-[(3-pyridinyloxy)methyl]cyclopropanamine,
their enantiomers, diastereoisomers, and also addition salts thereof with a pharmaceutically acceptable acid or base.

7. Process for the preparation of compounds of formula (I) according to claim 1, **characterised in that** there is used as starting material a compound of formula (II) : 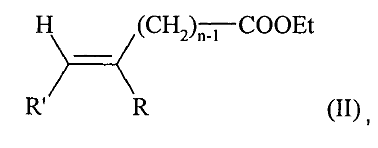 wherein R represents a group selected from ethoxycarbonyl, cyano and isocyano and R' represents a group selected from methyl and 2-bromopropyl, which compound of formula (II) is reacted with trimethylsulphoxonium iodide in the presence of sodium hydride in dimethyl sulphoxide to yield compounds of formula (III) : 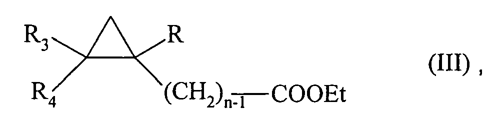 wherein R is as defined hereinabove and R₃, R₄ and n are as defined for formula (I),
which compounds of formula (III) are :
- either, when R represents an ethoxycarbonyl group, placed in the presence of sodium hydroxide in ethanol to yield compounds of formula (IV) : 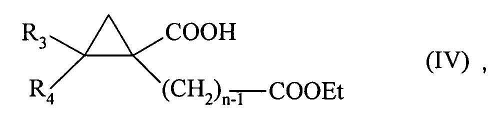
wherein R₃, R₄ and n are as defined hereinabove,
which compounds of formula (IV) are reacted with diphenylphosphoryl azide and *tert-*butanol in toluene, in the presence of triethylamine, to yield compounds of formula (V) : 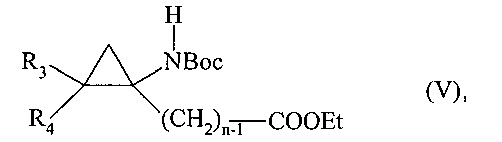 wherein R₃, R₄ and n are as defined hereinabove and Boc represents a *tert*-butoxycarbonyl group, which compounds of formula (V) are reacted with a compound of formula (VI) :
R'₁-L₁ (VI),
wherein R'₁ represents a group selected from linear or branched (C₁-C₆)alkyl and aryl-(C₁-C₆)alkyl in which the alkyl moiety may be linear or branched and L₁ represents a leaving group customary in organic chemistry, in the presence of potassium *tert*-butanolate in dimethylformamide, to yield compounds of formula (VII) : 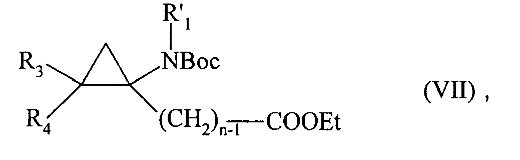 wherein R₃, R₄, R'₁, Boc and n are as defined hereinabove,
the totality of the compounds of formula (V) and (VII) constituting the compounds of formula (VIII) : 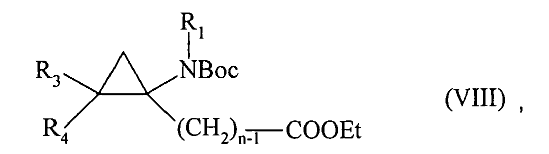 wherein R₃, R₄, Boc and n are as defined hereinabove and R₁ is as defined for formula (I), which compounds of formula (VIII) are :
* either placed in the presence of a reducing agent in an anhydrous solvent to yield compounds of formula (IX) : 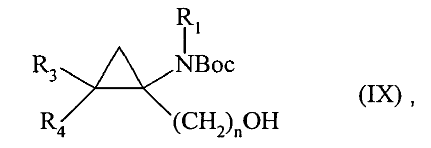
wherein R₃, R₄, Boc, n and R₁ are as defined hereinabove,
which compounds of formula (IX) are either reacted with triphenylphosphine and tetrabromomethane in ether to yield compounds of formula (X) : 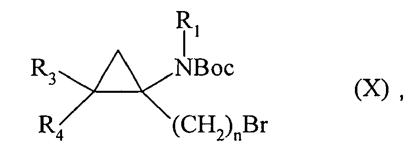 wherein R₃, R₄, Boc, n and R₁ are as defined hereinabove,
which compounds of formula (X) are reacted with a compound of formula (XI) : 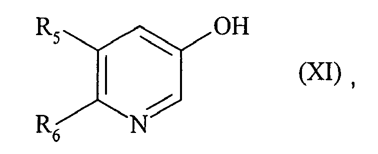 wherein R₅ and R₆ are as defined for formula (I), in the presence of caesium carbonate in butanone, to yield compounds of formula (XII) : 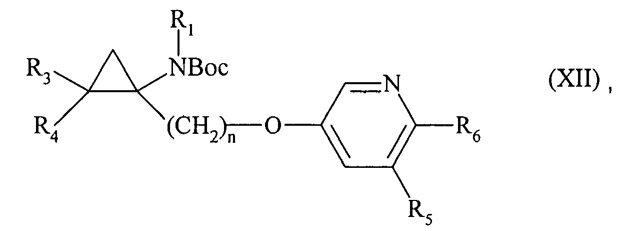 wherein R₃, R₄, R₅, R₆, R₁, n and Boc are as defined hereinabove,
which compounds of formula (XII) are placed in the presence of hydrochloric acid in dioxane to yield compounds of formula (I/a), a particular case of the compounds of formula (I) : 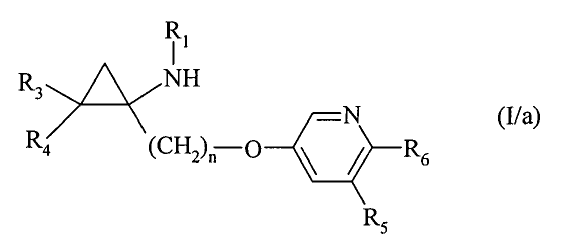 wherein R₁, R₃, R₄, R₅, R₆ and n are as defined hereinabove,
which compounds of formula (I/a) are:
- either treated with formic acid and an aqueous formaldehyde solution to yield compounds of formula (I/b), a particular case of the compounds of formula (I) : 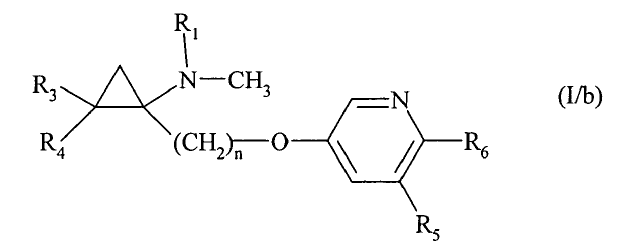
wherein R₁, R₃, R₄, R₅, R₆ and n are as defined hereinabove,
- or reacted with a compound of formula (XIII) :
R'₂-L₁ (XIII),
wherein L₁ is as defined hereinabove and R'₂ represents a group selected from linear or branched (C₁-C₆)alkyl and aryl-(C₁-C₆)alkyl in which the alkyl moiety may be linear or branched, under the same conditions as the compounds of formula (V), to yield compounds of formula (I/c), a particular case of the compounds of formula (I) : 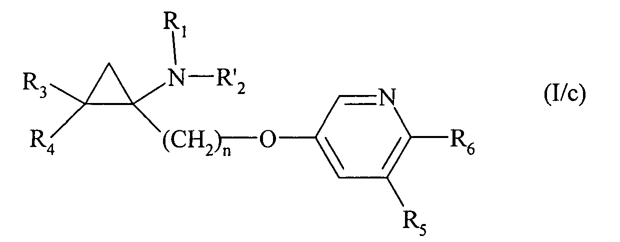 wherein R₁, R₃, R₄, R₅, R₆, n and R'₂ are as defined hereinabove,
or which compounds of formula (VIII) are :
* either treated with formic acid and an aqueous formaldehyde solution to yield compounds of formula (XIV) : 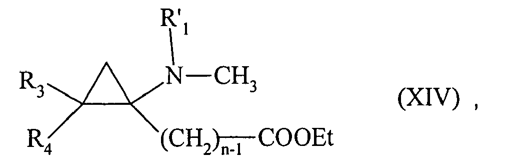
wherein R'₁, R₃, R₄ and n are as defined hereinabove,
* or treated, in succession, :
- with hydrochloric acid in dioxane
- and then with a compound of formula (XIII) as defined hereinabove, under the same conditions as compounds of formula (V), to yield compounds of formula (XV) : 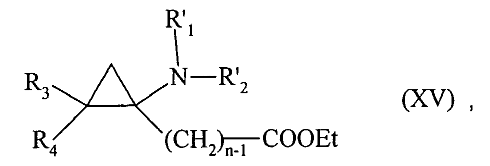
wherein R'₁, R'₂, R₃, R₄ and n are as defined hereinabove,
which compounds of formula (XIV) and (XV) are placed in the presence of a reducing agent in an anhydrous solvent to yield compounds of formula (XVI) : 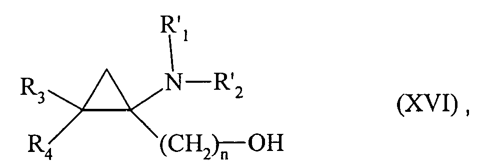 wherein R'₁, R'₂, R₃, R₄ and n are as defined hereinabove,
which compounds of formula (XVI) are reacted with a compound of formula (XVII) : 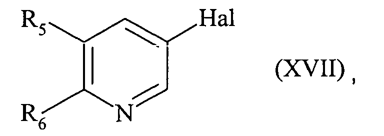 wherein R₅ and R₆ are as defined hereinabove and Hal represents a bromine or fluorine atom, in the presence of potassium *tert*-butanolate in DMSO, to yield compounds of formula (I/d), a particular case of the compounds of formula (I) : 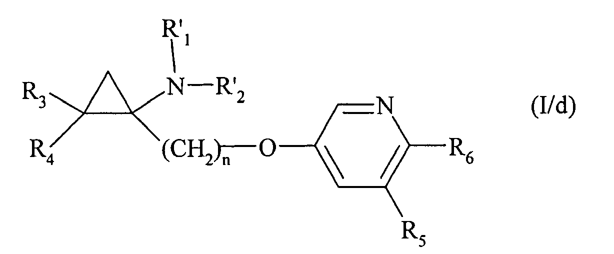 wherein R'₁, R'₂, R₃, R₄, R₅, R₆ and n are as defined hereinabove,
which compounds of formula (I/d), when R'₁ represents a benzyl group, are reacted with hydrochloric acid in the presence of palladium hydroxide in ethanol to yield compounds of formula (I/e), a particular case of the compounds of formula (I) : 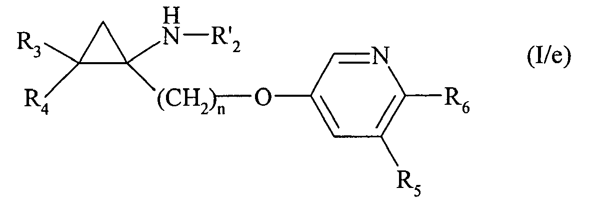 wherein R'₂, R₃, R₄, R₅, R₆ and n are as defined hereinabove,
or which compounds of formula (VIII) are :
* treated, in succession, :
- with hydrochloric acid in ethanol,
- and then with aqueous sodium hydrogen carbonate solution, to yield compounds of formula (XVIII) : 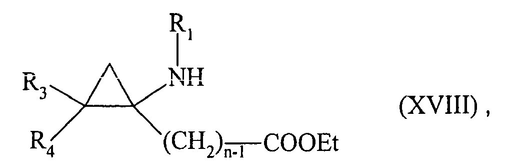
wherein R₁, R₃, R₄ and n are as defined hereinabove,
which compounds of formula (XVIII) are reacted with benzoyl chloride, in the presence of triethylamine in tetrahydrofuran, to yield compounds of formula (XIX) : 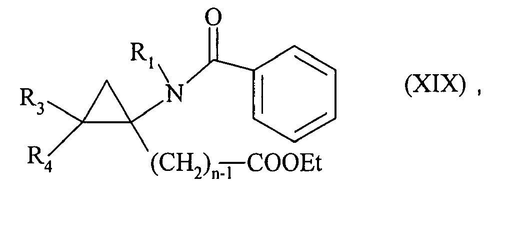 wherein R₁, R₃, R₄ and n are as defined hereinabove,
which compounds of formula (XIX) are placed in the presence of a reducing agent in an anhydrous solvent to yield compounds of formula (XX) : 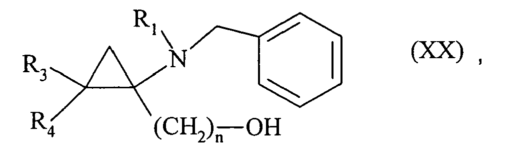 wherein R₁, R₃, R₄ and n are as defined hereinabove,
which compounds of formula (XX) are subjected to the same reaction conditions as the compounds of formula (XVI) to yield compounds of formula (I/f), a particular case of the compounds of formula (I) : 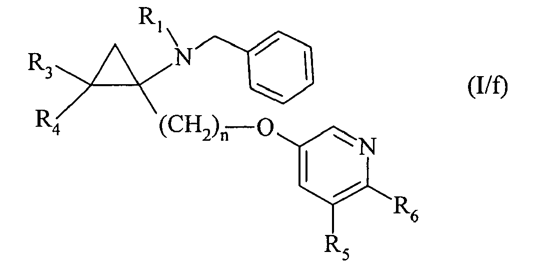 wherein R₁, R₃, R₄, R₅, R₆ and n are as defined hereinabove,
which compounds of formula (I/f) are subjected to the same reactions conditions as the compounds of formula (I/d) to yield compounds of formula (I/a) as defined hereinabove, a particular case of the compounds of formula (I),
or which compounds of formula (IX) are :
- when R₁ represents a hydrogen atom, reacted with hydrochloric acid in dioxanne to yield compounds of formula (XXI) : 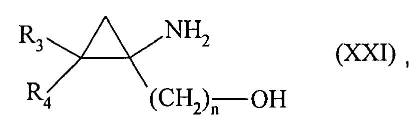
wherein R₃, R₄ and n are as defined hereinabove,
which compounds of formula (XXI) are subjected to the same reactions conditions as the compounds of formula (XVI) to yield compounds of formula (I/g), a particular case of the compounds of formula (I), the compounds of formula (I/g) having cis stereochemistry of (CH₂)ₙ in relation to one of the substituents R₃ or R₄, which represents an alkyl radical, while the other substituent R₃ or R₄ represents a hydrogen atom : 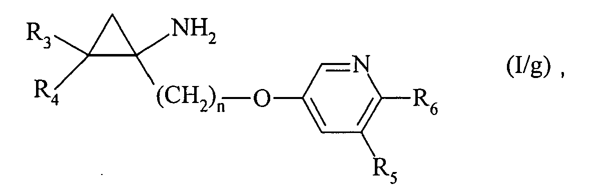 wherein R₃, R₄, R₅, R₆ and n are as defined hereinabove,
or which compounds of formula (III) are :
- either, when R represents an isocyano group, reacted with lithium aluminium hydride in ether to yield compounds of formula (XXII) : 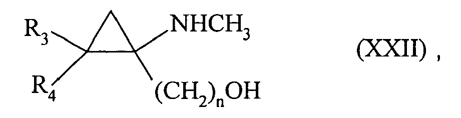
wherein R₃, R₄ and n are as defined hereinabove,
which compounds of formula (XXII) are reacted with a compound of formula (XVII) as defined hereinabove, under the same conditions as the compounds of formula (XVI), to yield compounds of formula (I/h), a particular case of the compounds of formula (I) : 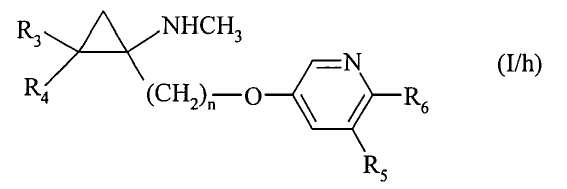 wherein R₃, R₄, R₅, R₆ and n are as defined hereinabove,
- or, when R represents a cyano group, reacted with sodium borohydride in tetrahydrofuran to yield compounds of formula (XXIII) : 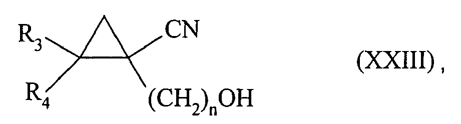
wherein R₃, R₄ and n are as defined hereinabove,
which compounds of formula (XXIII) are subjected to the same reaction conditions as the compounds of formula (XVI) to yield compounds of formula (XXIV) : 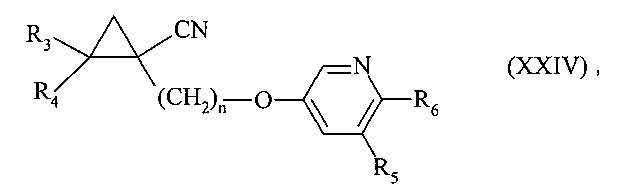 wherein R₃, R₄, R₅, R₆ and n are as defined hereinabove,
which compounds of formula (XXIV) are placed in the presence of an aqueous solution of hydrogen peroxide and lithium hydroxide in ethanol to yield compounds of formula (XXV) : 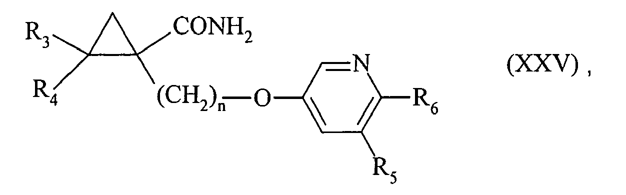 wherein R₃, R₄, R₅, R₆ and n are as defined hereinabove,
which compounds of formula (XXV) are reacted with bromine and potassium hydroxide in water to yield compounds of formula (I/i), a particular case of the compounds of formula (I), the compounds of formula (I/i) having cis stereochemistry of NH₂ in relation to one of the substituents R₃ or R₄, which represents an alkyl radical, while the other substituent R₃ or R₄ represents a hydrogen atom : 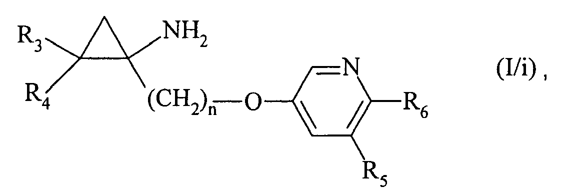 wherein R₃, R₄, R₅, R₆ and n are as defined hereinabove,
which compounds of formula (I/i) are subjected to the same reactions conditions as the compounds of formula (I/a) to yield compounds of formula (I/j), a particular case of the compounds of formula (I) : 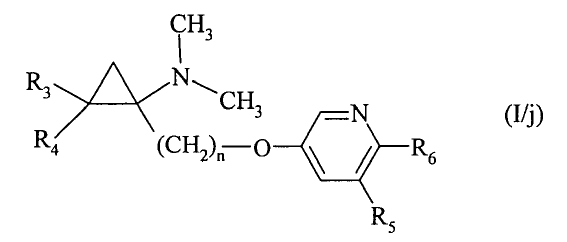 wherein R₃, R₄, R₅, R₆ and n are as defined hereinabove,
the totality of the compounds of formula (I/a) to (I/j) constituting the totality of the compounds of the invention, which are purified, where appropriate, according to conventional purification techniques, which may be separated into their different isomers according to a conventional separation technique, and which are converted, where appropriate, into addition salts thereof with a pharmaceutically acceptable acid or base.

8. Pharmaceutical compositions comprising as active ingredient at least one compound according to any one of claims 1 to 6, alone or in combination with one or more pharmaceutically acceptable, inert, non-toxic excipients or carriers.

9. Pharmaceutical compositions according to claim 8 comprising at least one active ingredient according to any one of claims 1 to 6 for use in the treatment of deficiencies of memory associated with cerebral ageing and with neurodegenerative diseases, and also for the treatment of mood disorders, Tourette's syndrome, attention-deficit hyperactivity syndrome, tobacco withdrawal and pain.

10. Pharmaceutical compositions according to claim 8 comprising at least one active ingredient according to any one of claims 1 to 6 for use in the treatment of deficiencies of memory associated with Alzheimer's disease, Parkinson's disease, Pick's disease, Korsakoff's disease or frontal lobe and subcortical dementias.

## Patentansprüche

1. Verbindungen der Formel (I): 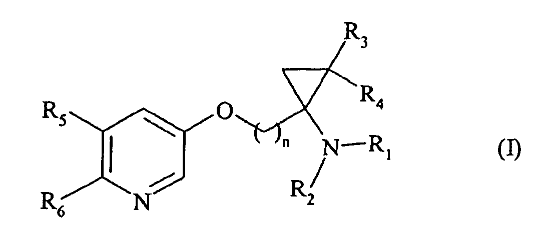 in der:
n eine ganze Zahl zwischen 1 und 6 einschließlich bedeutet,
R₁ und R₂, die gleichartig oder verschieden sind, unabhängig voneinander ein Wasserstoffatom oder eine geradkettige oder verzweigte (C₁-C₆)-Alkylgruppe oder eine geradkettige oder verzweigte Aryl-(C₁-C₆)-alkylgruppe bedeuten.
R₃ und R₄, die gleichartig oder verschieden sind, unabhängig voneinander ein Wasserstoffatom oder eine geradkettige oder verzweigte (C₁-C₆)-Alkylgruppe bedeuten, mit der Maßgabe, dass mindestens eine der Gruppen R₃ oder R₄ ei-ne geradkettige oder verzweigte (C₁-C₆-Alkylgruppe darstellt, und
R₅ und R₆, die gleichartig oder verschieden sind, unabhängig voneinander ein Wasserstoffatom oder eine geradkettige oder verzweigte (C₁-C₆)-Alkylgruppe, ein Halogenatom, eine Hydroxygruppe, eine geradkettige oder verzweigte (C₁-C₆)-Alkoxygruppe, Cyanogruppe, Nitrogruppe, geradkettige oder verzweigte (C₁-C₆)-Acylgruppe, geradkettige oder verzweigte (C₁-C₆)-Alkoxycarbonyl-gruppe, geradkettige oder verzweigte (C₁-C₆)-Trihalogenalkylgruppe, geradket-tige oder verzweigte (C₁-C₆)-Trihalogenalkoxygruppe oder eine Aminogruppe, die gegebenenfalls durch eine oder zwei geradkettige oder verzweigte (C₁-C₆)-Alkylgruppe substituiert ist, bedeuten,
wobei man unter einer Arylgruppe eine Phenyl-, Biphenyl-, Naphthyl-, Dihydronaphthyl-, Tetrahydronaphthyl-, Indanyl- und Indenyl-gruppe versteht, wobei jede dieser Gruppen gegebenenfalls durch eine oder mehrere gleichartige oder verschiedenartige Gruppe substituiert ist, ausgewählt aus Halogen, geradkettigem oder verzweigtem (C₁-C₆)-Alkyl, Hydroxy, Cyano, Nitro, geradkettigem oder verzweigtem (C₁-C₆)-Alkoxy, geradkettigem oder verzweigtem (C₂-C₇)-Acyl, geradkettigem oder verzweigtem (C₁-C₆)-Alkoxycarbonyl, geradkettigem oder verzweigtem (C₁-C₆)-Trihalogenalkyl, geradkettigem oder verzweigtem (C₁-C₆)-Trihalogenalkoxy und Amino, welches gegebenenfalls durch eine oder zwei geradkettige oder verzweigte (C₁-C₆)-Alkylgruppen substituiert ist, deren Enantiomere, Diastereoisomere sowie deren Additionssalze mit einer pharmazeutisch annehmbaren Säure oder Base.

2. Verbindungen der Formel (I) nach Anspruch 1, **dadurch gekennzeichnet, dass** n eine ganze Zahl mit einem Wert von 1 ist, deren Enantiomere. Diastereoisomere sowie deren Additionssalze mit einer pharmazeutisch annehmbaren Säure oder Base.

3. Verbindungen der Formel (I) nach Anspruch 1, **dadurch gekennzeichnet, dass** R₁ und R₂, die gleichartig oder verschieden sind, jeweils ein Wasserstoffatom oder eine geradkettige oder verzweigte (C₁-C₆)-Alkylgruppe bedeuten, deren Enantiomere, Diastereoisomere sowie deren Additionssalze mit einer pharmazeutisch annehmbaren Säure oder Base.

4. Verbindungen der Formel (I) nach Anspruch 1, **dadurch gekennzeichnet, dass** R₃ und R₄, die gleichartig oder verschieden sind, jeweils ein Wasserstoffatom oder eine Methylgruppe bedeuten, deren Enantiomere, Diastereoisomere sowie deren Additionssalze mit einer pharmazeutisch annehmbaren Säure oder Base.

5. Verbindungen der Formel (I) nach Anspruch 1, **dadurch gekennzeichnet, dass** R₅ und R₆, die gleichartig oder verschieden sind, jeweils ein Wasserstoffatom oder ein Halogenatom bedeuten, deren Enantiomere, Diastereoisomere sowie deren Additionssalze mit einer pharmazeutisch annehmbaren Säure oder Base.

6. Verbindungen der Formel (I) nach Anspruch 1, nämlich:
(1*S*,2*S*),(1*R*,2*R*)-*N*,2-Dimethyl-1-[(3-pyridinyloxy)-methyl]-cyclopropanamin,
(1*S*,2*S*) oder (1*R*,2*R*)-(-)-*N*,2-Dimethyl-1-[(3-pyridinyloxy)-methyl]-cyclopropan-amin,
(1*S*,2*S*) oder (1*R*,2*R*)-(+)-*N*,2-Dimethyl-1-[(3-pyridinyloxy)-methyl]-cyclopropan-amin,
(1*S*,2*S*),(1*R*,2*R*)-2-Methyl-1-[(3-pyridinyloxy)-methyl]-cyclopropanamin,
(1*S*,2*S*) oder (1*R*,2*R*)-(-)-2-Methyl-1-[(3-pyridinyloxy)-methyl]-cyclopropanamin,
(1*S*,2*S*) oder (1*R*,2*R*)-(+)-2-Methyl-1-[(3-pyridinyloxy)-methyl]-cyclopropanamin,
(1*S*,2*S*),(1*R*,2*R*)-*N*,*N*,2-Trimethyl-1-[(3-pyridinyloxy)-methyl]-cyclopropanamin,
(1*S*,2*S*) oder (1*R*,2*R*)-(+)-*N*,*N*,2-Trimethyl-1-[(3-pyridinyloxy)-methyl]-cyclopropan-amin,
(1*S*,2*S*) oder (1*R*,2*R*)-(-)-*N*,*N*,2-Trimethyl-1-[(3-pyridinyloxy)-methyl]-cyclopropan-amin,
(1*S*,2*S*),(1*R*,2*R*)-1-{[(6-Chlor-3-pyridinyl)-oxy]-methyl}-*N*,2-dimethylcyclopropan-amin.
(1*S*,2*S*) oder (1*R*,2*R*)-(-)1-{[(6-Chlor-3-pyridinyl)-oxy]-methyl}-*N*,2-dimethylcyclo-propanamin,
(1*S*,2*S*) oder (1*R*,2*R*)-(+)1-{[(6-Chlor-3-pyridinyl)-oxy]-methyl}-*N*,2-dimethylcyclo-propanamin.
(1*S*,2*S*),(1*R*,2*R*)-1-{[(6-Chlor-3-pyridinyl)-oxy]-methyl}-2-methylcyclopropanamin,
(1*S*,2*S*) oder (1*R*,2*R*)-(-)1-{[(6-Chlor-3-pyridinyl)-oxy]-methyl}-2-methylcyclopropan-amin,
(1*S*,2*S*) oder (1*R*,2*R*)-(+)1-{[(6-Chlor-3-pyridinyl)-oxy]-methyl}-2-methylcyclopro-panamin,
(1*S*,2*S*),(1*R*,2*R*)-1-{[(6-Chlor-3-pyridinyl)-oxy]-methyl}-*N*,*N*,2-trimethylyclopropan-amin,
(1*S*,2*S*) oder (1*R*,2*R*)-(+)-1-{[(6-Chlor-3-pyridinyl)-oxy]-methyl}-*N*,*N*,2-trimethyl-cyclopropanamin,
(1*S*,2*S*) oder (1*R*,2*R*)-(-)-1-{[(6-Chlor-3-pyridinyl)-oxy]-methyl}-*N*,*N*,2-trimethyl-cyclopropanamin,
(1*S*,2*R*),(1*R*,2*S*)-*N*,2-Dimethyl-1-[(3-pyridinyloxy)-methyl]-cyclopropanamin,
(1*S*,2*R*) oder (1*R*,2*S*)-(+)-*N*,2-Dimethyl-1-[(3-pyridinyloxy)-methyl]-cyclopropan-amin,
(1*S*,2*R*) oder (1*R*,2*S*)-(-)-*N*,2-Dimethyl-1-[(3-pyridinyloxy)-methyl]-cyclopropan-amin,
(1*S*,2*R*),(1*R*,2*S*)-1-{[(5-Brom-3-pyridinyl)-oxy]-methyl}-*N*,2-dimethylcyclopropan-amin,
(1*S*,2*R*),(1*R*,2*S*)-2-Methyl-1-[(3-pyridinyloxy)-methyl]-cyclopropanamin,
(1*S*,2*R*),(1*R*,2*S*)-*N*,*N*,2-Trimethyl-1-[(3-pyridinyloxy)-methyl]-cyclopropanamin,
(*R*,*S*)-*N*,2,2-Trimethyl-1-[(3-pyridinyloxy)-methyl]-cyclopropanamin,
(R oder *S*)-(+)-*N*,2,2-Trimethyl-1-[(3-pyridinyloxy)-methyl]-cyclopropanamin,
(R oder *S*)-(-)-*N*,2,2-Trimethyl-1-[(3-pyridinyloxy)-methyl]-cyclopropanamin,
deren Enantiomere, Diastereoisomere sowie deren Additionssalze mit einer pharmazeutisch annehmbaren Säure oder Base.

7. Verfahren zur Herstellung der Verbindungen der Formel (I) nach Anspruch 1, **dadurch gekennzeichnet, dass** man als Ausgangsprodukt eine Verbindung der Formel (II) verwendet: 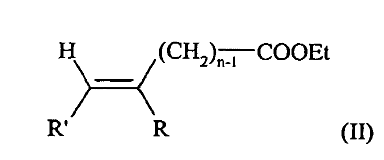 in der R eine Gruppe bedeutet ausgewählt aus Ethoxycarbonyl, Cyano und Isocyano, R' eine Gruppe bedeutet ausgewählt aus Methyl und 2-Brompropyl, welche Verbindung der Formel (II) in Gegenwart von Natriumhydrid in Dimethylsulfoxid mit Trimethylsulfoxoniumiodid umgesetzt wird zur Bildung der Verbindungen der Formel (III): 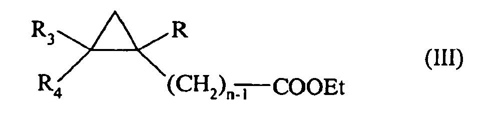 in der R die oben angegebenen Bedeutungen besitzt und R₃, R₄ und n die bezüglich der Formel (I) angegebenen Bedeutungen aufweisen,
welche Verbindungen der Formel (III):
- entweder, wenn R eine Ethoxycarbonylgruppe bedeutet, mit Natriumhydroxid in Ethanol umgesetzt werden zur Bildung der Verbindungen der Formel (IV): 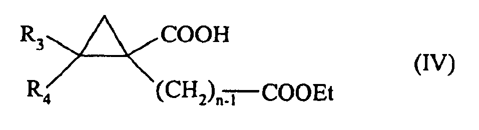
in der R₃, R₄ und n die oben angegebenen Bedeutungen besitzen,
welche Verbindungen der Formel (IV) in Gegenwart von Triethylamin mit Diphenylphosphorylazid und tert.-Butanol in Toluol umgesetzt werden zur Bildung der Verbindungen der Formel (V): 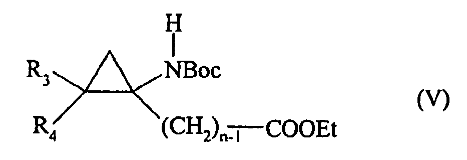 in der R₃, R₄ und n die oben angegebenen Bedeutungen besitzen und Boc die tert.-Butoxycarbonylgruppe darstellt, welche Verbindungen der Formel (V) mit einer Verbindung der Formel (VI):
R'₁ - L₁ (VI)
in der R'₁ eine Gruppe bedeutet ausgewählt aus geradkettigem oder verzweigtem (C₁-C₆)-Alkyl und geradkettigem oder verzweigtem Aryl-(C₁-C₆)-alkyl und L₁ eine in der organischen Chemie übliche austretende Gruppe darstellt, in Gegenwart von Kalium-tert.-butylat in Dimethylformamid umgesetzt werden zur Bildung der Verbindungen der Formel (VII): 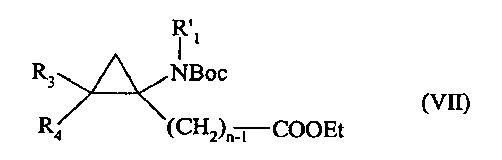 in der R₃, R₄, R'₁, Boc und n die oben angegebenen Bedeutungen besitzen,
wobei die Gesamtheit der Verbindungen der Formeln (V) und (VII) die Verbindungen der Formel (VIII) bilden: 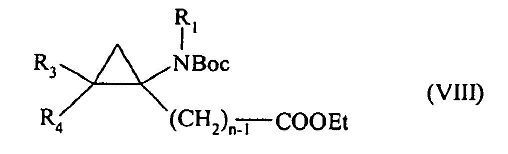 in der R₃, R₄, Boc und n die oben angegebenen Bedeutungen besitzen und R₁ die bezüglich der Formel (I) angegebenen Bedeutungen besitzt,
welche Verbindungen der Formel (VIII):
* entweder in Gegenwart eines Reduktionsmittels in einem wasserfreiem Lösungsmittel umgesetzt werden zur Bildung der Verbindungen der Formel (IX): 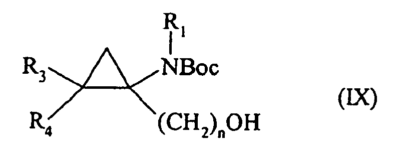
in der R₃, R₄, Boc, n und R₁ die oben angegebenen Bedeutungen besitzen,
welche Verbindungen der Formel (IX) mit Triphenylphosphin und Tetrabrommethan in Ether umgestzt werden zur Bildung der Verbindungen der Formel (X): 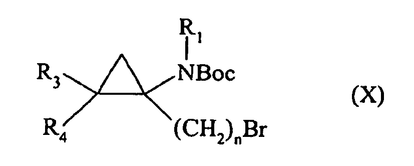 in der R₃, R₄, Boc, n und R₁ die oben angegebenen Bedeutungen besitzen,
welche Verbindungen der Formel (X) mit einer Verbindung der Formel (XI): 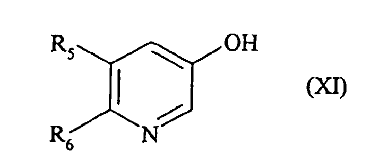 in der R₅ und R₆ die bezüglich der Formel (I) angegebenen Bedeutungen besitzen, in Gegenwart von Cäsiumcarbonat in Butanon umgesetzt werden zur Bildung der Verbindungen der Formel (XII): 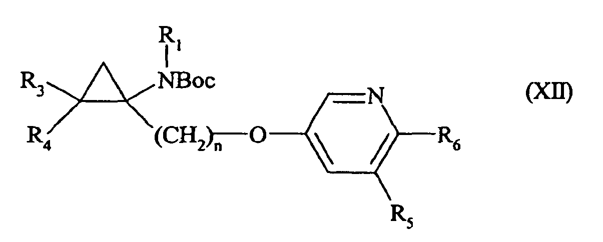 in der R₃, R₄, R₅, R₆, R₁, n und Boc die oben angegebenen Bedeutungen besitzen,
welche Verbindungen der Formel (XII) mit Chlorwasserstoffsäure in Dioxan umgesetzt werden zur Bildung der Verbindungen der Formel (I/a), einem Sonderfall der Verbindungen der Formel (I): 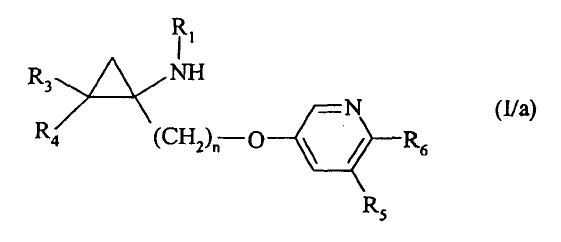 in der R₁, R₃, R₄, R₅, R₆ und n die oben angegebenen Bedeutungen besitzen,
welche Verbindungen der Formel (I/a):
- entweder mit Ameisensäure und einer wässrigen Formaldehydlösung umgesetzt werden zur Bildung der Verbindungen der Formel (I/b), einem Sonderfall der Verbindungen der Formel (I): 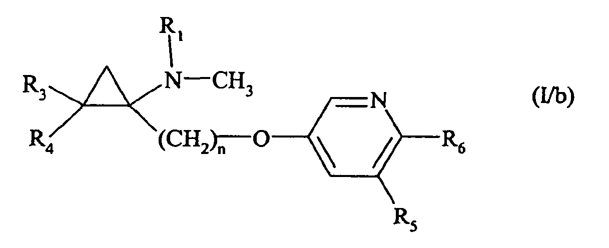 in der R₁, R₃, R₄, R₅, R₆ und n die oben angegebenen Bedeutungen besitzen,
- oder mit einer Verbindung der Formel (XIII):
R'₂ - L₁ (XIII)
in der L₁ die oben angegebenen Bedeutungen besitzt und R'₂ eine Gruppe bedeutet ausgewählt aus geradkettigem oder verzweigtem (C₁-C₆)-Alkyl und geradkettigem oder verzweigtem Aryl-(C₁-C₆)-alkyl unter den gleichen Bedingungen wie die Verbindungen der Formel (V) umgesetzt werden zur Bildung der Verbindungen der Formel (I/c), einem Sonderfall der Verbindungen der Formel (I): 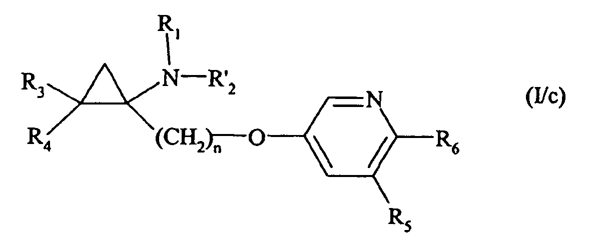 in der R₁, R₃, R₄, R₅, R₆, n und R'₂ die oben angegebenen Bedeutungen besitzen,
oder die Verbindungen der Formel (VIII):
* entweder mit Ameisensäure in einer wässrigen Formaldehydlösung umgesetzt werden zur Bildung der Verbindungen der Formel (XIV): 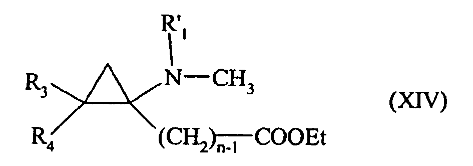
in der R'₁, R₃, R₄ und n die oben angegebenen Bedeutungen besitzen,
* oder nacheinander:
- mit Chlorwasserstoffsäure in Dioxan behandelt werden,
- dann mit einer Verbindung der Formel (XIII), wie sie oben definiert worden ist, unter den gleichen Bedingungen wie die Verbindungen der Formel (V) umgesetzt werden zur Bildung der Verbindungen der Formel (XV): 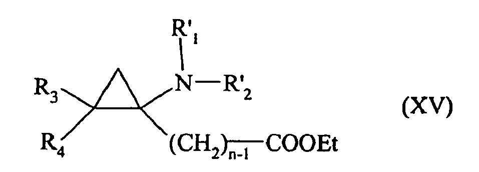
in der R'₁, R'₂, R₃, R₄ und n die oben angegebenen Bedeutungen besitzen,
welche Verbindungen der Formeln (XIV) und (XV) mit einem Reduktionsmittel in einem wasserfreien Lösungsmittel umgesetzt werden zur Bildung der Verbindungen der Formel (XVI): 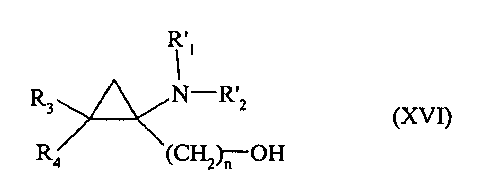 in der R'₁, R'₂, R₃, R₄ und n die oben angegebenen Bedeutungen besitzen,
welche Verbindungen der Formel (XVI) mit einer Verbindung der Formel (XVII): 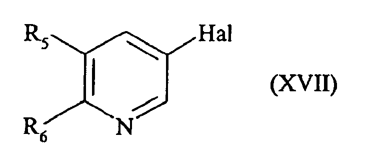 in der R₅ und R₆ die oben angegebenen Bedeutungen besitzen und Hal ein Brom-oder Fluoratom bedeutet, in Gegenwart von Kalium-tert.-butylat in DMSO umgesetzt werden zur Bildung der Verbindungen der Formel (I/d), einem Sonderfall der Verbindungen der Formel (I): 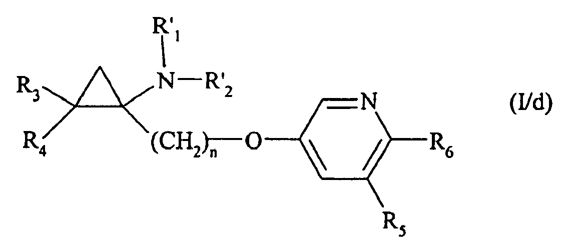 in der R'₁, R'₂, R₃, R₄, R₅, R₆ und n die oben angegebenen Bedeutungen besitzen,
welche Verbindungen der Formel (I/d), wenn R'₁ eine Benzylgruppe bedeutet, mit Chlorwasserstoffsäure in Gegenwart von Palladiumhydroxid in Ethanol umgesetzt werden zur Bildung der Verbindungen der Formel (I/e), einem Sonderfall der Verbindungen der Formel (I): 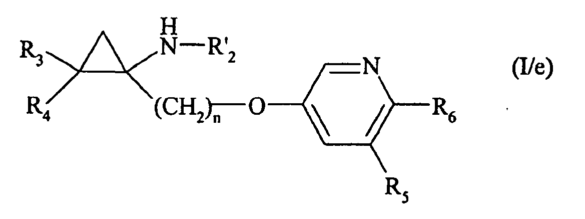 in der R'₂, R₃, R₄, R₅, R₆ und n die oben angegebenen Bedeutungen besitzen,
oder die Verbindungen der Formel (VIII):
* nacheinander:
- mit Chlorwasserstoffsäure in Ethanol behandelt werden,
- dann mit einer wässrigen Kaliumhydrogencarbonatlösung umgesetzt werden zur Bildung der Verbindungen der Formel (XVIII): 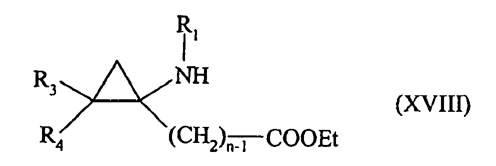
in der R₁, R₃, R₄ und n die oben angegebenen Bedeutungen besitzen,
welche Verbindungen der Formel (XVIII) mit Benzoylchlorid in Gegenwart von Triethylamin in Tetrahydrofuran umgesetzt werden zur Bildung der Verbindungen der Formel (XIX): 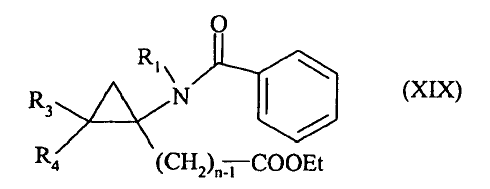 in der R₁, R₃, R₄ und n die oben angegebenen Bedeutungen besitzen,
welche Verbindungen der Formel (XIX) mit einem Reduktionsmittel in einem wasserfreien Lösungsmittel umgesetzt werden zur Bildung der Verbindungen der Formel (XX): 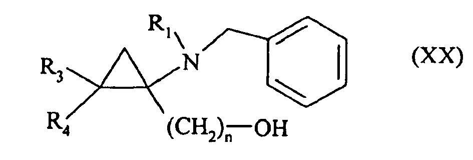 in der R₁, R₃, R₄ und n die oben angegebenen Bedeutungen besitzen,
welche Verbindungen der Formel (XX) unter den gleichen Reaktionsbedingungen wie die Verbindungen der Formel (XVI) umgesetzt werden zur Bildung der Verbindungen der Formel (I/f), einem Sonderfall der Verbindungen der Formel (I): 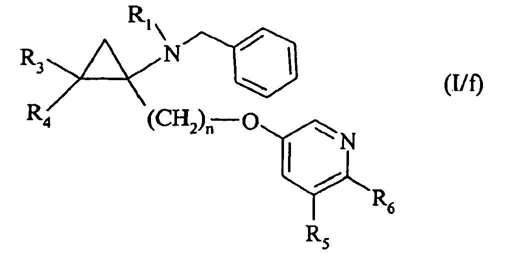 in der R₁, R₃, R₄, R₅, R₆ und n die oben angegebenen Bedeutungen besitzen,
welche Verbindungen der Formel (I/f) den gleichen Reaktionsbedingungen unter- 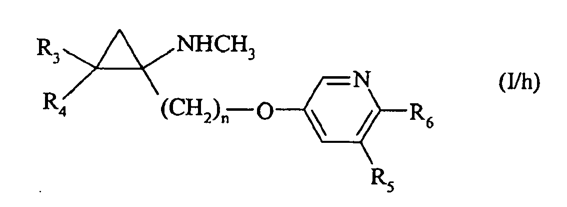 in der R₃, R₄, R₅, R₆ und n die oben angegebenen Bedeutungen besitzen,
- oder wenn R eine Cyanogruppe bedeutet, mit Natriumborhydrid in Tetrahydrofuran umgesetzt werden zur Bildung der Verbindungen der Formel (XXIII): 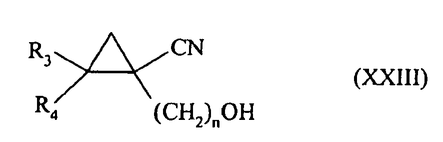
in der R₃, R₄ und n die oben angegebenen Bedeutungen besitzen,
welche Verbindungen der Formel (XXIII) den gleichen Reaktionsbedingungen unterworfen werden wie die Verbindungen der Formel (XVI) zur Bildung der Verbindungen der Formel (XXIV): 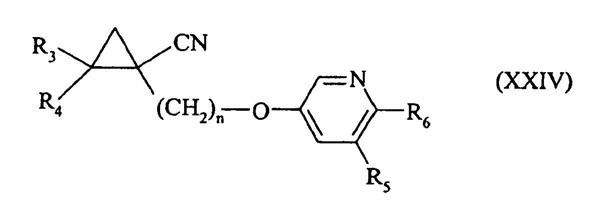 in der R₃, R₄, R₅, R₆ und n die oben angegebenen Bedeutungen besitzen,
welche Verbindungen der Formel (XXIV) mit einer wässrigen Wasserstoffperoxidlösung und Lithiumhydroxid in Ethanol umgesetzt werden zur Bildung der Verbindungen der Formel (XXV): 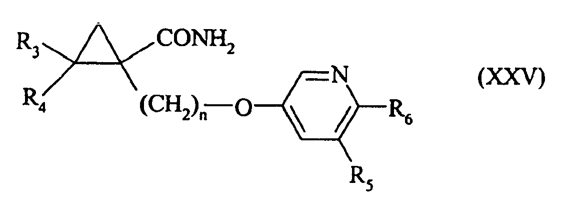 in der R₃, R₄, R₅, R₆ und n die oben angegebenen Bedeutungen besitzen,
welche Verbindungen der Formel (XXV) mit Brom und Kaliumhydroxid in Wasser umgesetzt werden zur Bildung der Verbindungen der Formel (I/i), einem Sonderfall der Verbindungen der Formel (I), wobei die Verbindungen der Formel (I/i) in der cis-stereochemischen Form von NH₂, bezogen auf einen der Substituenten R₃ oder R₄, der eine Alkylgruppe darstellt, während der andere Substituent R₃ oder R₄ ein Wasserstoffatom bedeutet, vorliegen: 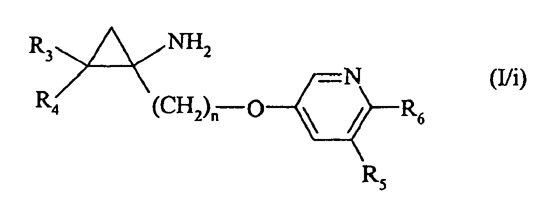 in der R₃, R₄, R₅, R₆ und n die oben angegebenen Bedeutungen besitzen,
welche Verbindungen der Formel (I/i) den gleichen Reaktionsbedingungen unterworfen werden wie die Verbindungen der Formel (I/a) zur Bildung der Verbindungen der Formel (I/j), einem Sonderfall der Verbindungen der Formel (I): 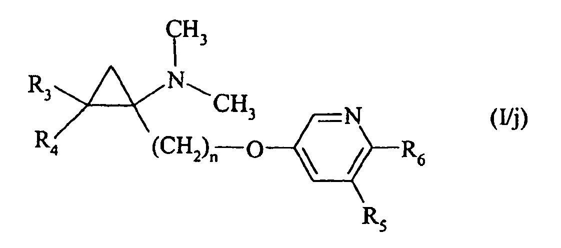 in der R₃, R₄, R₅, R₆ und n die oben angegebenen Bedeutungen besitzen,
wobei die Gesamtheit der Verbindungen der Formeln (I/a) bis (I/j), welche die Gesamtheit der erfindungsgemäßen Verbindungen bilden, gegebenenfalls mit Hilfe klassischer Reinigungsmethoden gereinigt werden, mit Hilfe einer klassischen Trennmethode in ihre verschiedenen Isomeren aufgetrennt werden können und gegebenenfalls in ihre Additionssalze mit einer pharmazeutisch annehmbaren Säure oder Base überführt werden können.

8. Pharmazeutische Zubereitungen enthaltend als Wirkstoff mindestens eine Verbindung nach einem der Ansprüche 1 bis 6 allein oder in Kombination mit einem oder mehreren inerten, nichttoxischen und pharmazeutisch annehmbaren Trägermaterialien oder Hilfsstoffen.

9. Pharmazeutische Zubereitungen nach Anspruch 8, enthaltend mindestens einen Wirkstoff nach einem der Ansprüche 1 bis 6, die nützlich sind zur Behandlung von Gedächtnisdefiziten, die mit dem Altern des Gehirns und neurodegenerativen Erkrankungen verknüpft sind, sowie zur Behandlung von Gemütsstörungen, des Tourette-Syndroms, des Hyperaktivitätssyndroms mit Aufmerksamkeitsdefiziten, für den Tabakentzug und von Schmerzen.

10. Pharmazeutische Zubereitungen nach Anspruch 8, enthaltend mindestens einen Wirkstoff nach einem der Ansprüche 1 bis 6, die nützlich sind bei der Behandlung von Gedächtnisdefiziten, die mit der Alzheimerschen Krankheit, der Parkinsonschen Krankheit, der Pickschen Krankheit, der Korsakoffschen Krankheit oder frontalen und subkortikalen Demenzen verknüpft sind.
